# EUROPEAN PATENT SPECIFICATION

(11) **EP 4 176 089 B1**
(45) Date of publication and mention of the grant of the patent: **10.09.2025**
(21) Application number: 21735737.5
(22) Date of filing: 02.07.2021
(51) Int. Cl.: C12Q 1/70

(54) **RAPID DETECTION KIT FOR HUMAN PATHOGENIC CORONAVIRUSES : BETACORONAVIRUS GROUP B/C AND SARS-COV-2**
SCHNELLNACHWEIS-KIT FÜR HUMANE PATHOGENE CORONAVIREN: BETACORONAVIRUS-GRUPPE B/C UND SARS-COV-2
KIT DE DÉTECTION RAPIDE POUR CORONAVIRUS PATHOGÈNES HUMAINS, BÊTACORONAVIRUS GROUPE B/C ET SRAS-COV-2

(30) Priority: 03.07.2020 EP 20305759
(43) Date of publication of application: 10.05.2023
(73) Proprietor: Institut Pasteur, 75724 Paris Cédex 15 (FR)
(72) Inventor: VANHOMWEGEN, Jessica, 75724 Paris Cedex 15 (FR); MANUGUERRA, Jean-Claude, 75724 Paris Cedex 15 (FR)
(74) Representative: Plasseraud IP
(86) International application number: PCT/EP2021/068409
(87) International publication number: WO 2022/003188

(56) References cited:
- CN-A- 111 321 249
- WEI E. HUANG ET AL: "RT-LAMP for rapid diagnosis of coronavirus SARS-CoV-2", MICROBIAL BIOTECHNOLOGY, vol. 13, no. 4, 25 April 2020 (2020-04-25), GB, pages 950 - 961, XP055733462, ISSN: 1751-7915, DOI: 10.1111/1751-7915.13586
- NN: "Executive Summary The Color SARS-CoV-2 LAMP Diagnostic Assay utilizes loop", 19 May 2020 (2020-05-19), XP055733468, Retrieved from the Internet <URL:https://www.color.com/wp-content/uploads/2020/05/LAMP-Diagnostic-Assay.pdf> [retrieved on 20200923]
- WEIHUA YANG ET AL: "Rapid Detection of SARS-CoV-2 Using Reverse transcription RT-LAMP method", MEDRXIV, 3 March 2020 (2020-03-03), XP055733463, Retrieved from the Internet <URL:https://www.medrxiv.org/content/10.1101/2020.03.02.20030130v2> [retrieved on 20200922], DOI: 10.1101/2020.03.02.20030130
- YUN HEE BAEK ET AL: "Development of a reverse transcription-loop-mediated isothermal amplification as a rapid early-detection method for novel SARS-CoV-2", EMERGING MICROBES & INFECTIONS, vol. 9, no. 1, 18 May 2020 (2020-05-18), pages 998 - 1007, XP055730864, DOI: 10.1080/22221751.2020.1756698
- YINHUA ZHANG ET AL: "Rapid Molecular Detection of SARS-CoV-2 (COVID-19) Virus RNA Using Colorimetric LAMP", MEDRXIV, 29 February 2020 (2020-02-29), XP055730127, Retrieved from the Internet <URL:https://www.medrxiv.org/content/10.1101/2020.02.26.20028373v1> [retrieved on 20200922], DOI: 10.1101/2020.02.26.20028373
- MINGHUA JIANG ET AL: "Development and Validation of a Rapid, Single-Step Reverse Transcriptase Loop-Mediated Isothermal Amplification (RT-LAMP) System Potentially to Be Used for Reliable and High-Throughput Screening of COVID-19", FRONTIERS IN CELLULAR AND INFECTION MICROBIOLOGY, vol. 10, 16 June 2020 (2020-06-16), XP055733475, DOI: 10.3389/fcimb.2020.00331
- LIN YU ET AL: "Rapid colorimetric detection of COVID-19 coronavirus using a reverse tran-scriptional loop-mediated isothermal amplification (RT-LAMP) diagnostic plat-form: iLACO", MEDRXIV, 24 February 2020 (2020-02-24), XP055730128, Retrieved from the Internet <URL:https://www.medrxiv.org/content/10.1101/2020.02.20.20025874v1> [retrieved on 20200922], DOI: 10.1101/2020.02.20.20025874
- LU RENFEI ET AL: "Development of a Novel Reverse Transcription Loop-Mediated Isothermal Amplification Method for Rapid Detection of SARS-CoV-2", VIROLOGICA SINICA, SPRINGER, DE, vol. 35, no. 3, 1 April 2020 (2020-04-01), pages 344 - 347, XP037234302, ISSN: 1674-0769, [retrieved on 20200401], DOI: 10.1007/S12250-020-00218-1
- C. YAN ET AL: "Rapid and visual detection of 2019 novel coronavirus (SARS-CoV-2) by a reverse transcription loop-mediated isothermal amplification assay", CLINICAL MICROBIOLOGY AND INFECTION., vol. 26, no. 6, 8 April 2020 (2020-04-08), United Kingdom, Switzerland, pages 773 - 779, XP055760335, ISSN: 1198-743X, DOI: 10.1016/j.cmi.2020.04.001

## Description

### FIELD

The disclosure relates to reagents and methods for the rapid detection of the presence and/or absence of SARS-CoV-2 and other betacoronavirus group B/C nucleic acid in a sample and their use for the diagnosis of an infection or the detection of an environmental contamination by SARS-CoV-2 and other betacoronavirus group B/C.

### BACKGROUND

In 2019, a new coronavirus named SARS-CoV-2 which causes the disease COVID-19 was isolated in association with cases of severe acute respiratory syndrome named (Liu et al., Viruses, 2020 Jan 22;12(2)).

Coronaviruses are enveloped, positive-sense, single stranded RNA viruses that infect humans and mammals. The betacoronavirus genus (Beta-CoV or β-CoV) which is divided in 4 lineages or groups (A, B, C, D) comprise the highly human-pathogenic coronaviruses in group B/C. Beta-CoV group B/C include SARS-Cov-2, SARS-Cov that emerged in China in 2002 (Group B) and the Middle East respiratory syndrome coronavirus (MERS-CoV), first detected in Saudi Arabia in 2012 (Group C; Zaki et al., N Engl J Med., 2012 Nov 8;367(19):1814-20 ; Lee et al., BMC Infect Dis. 2017 Jul 14;17(1):498). In contrast, Beta-CoV group A include HCoV-OC43 and HCoV-HKU1 which can cause the common cold.

SARS-Cov-2 genome comprises 6-11 open reading frames (ORFs) encoding a large polyprotein. The first ORF (ORF1a/b) which comprises a large portion of the genome encodes 16 nonstructural proteins (nsp) including papain-like protease (nsp3), RNA-dependent-RNA polymerase (RdRp or nsp12) and others likely to be involved in the transcription and replication of SARS-Cov-2. In addition to nsps, the genome encodes four major structural proteins including spike surface glycoprotein (S), membrane glycoprotein (M), nucleocapsid protein (N), envelope E and accessory protein like ORFs;

COVID-19 is difficult to differentiate clinically from the various etiologic agents of respiratory illnesses. Inexpensive, portable, and easy-to-use diagnostic devices are therefore urgently needed to ensure rapid detection of COVID-19 at the point-of-care. Unfortunately, the availability of laboratoty diagnosis in high disease-burden and low-resource areas is currently limited by cost, infrastructure, and personnel constraints. Rapid nucleic acid testing for viral diseases can provide access to much-needed diagnostic methods, especially for applications requiring fast turnaround times Among field-applicable isothermal nucleic acid amplification methods developed for rapid, simple, and cost-effective detection of pathogenic microorganisms in smaller size systems, the RT-LAMP (reverse-transcription loop-mediated isothermal amplification) appears among the most promising assays, highly suited for on-site detection of SARS-CoV-2 infections. The RT-LAMP assay uses a DNA polymerization enzyme with high strand-displacement activity and 6 primers, specifically designed to recognize 8 distinct regions on the target gene, to synthesize large amounts of target viral nucleic acids under a constant temperature (65°C). The accelerated RT-LAMP reaction yields high amount of amplification products, which can be detected in real time with simple detectors either by: (i) fluorescence, using DNA intercaling dyes; (ii) colorimetry, using pH indicators or (iii) turbidity, as the reaction produces large amounts of magnesium pyrophosphate (a white precipitate). In addition to its relative simplicity and low infrastructure costs, the RT-LAMP assay (i) has moderate incubation temperature leading to simplified heating and low power consumption, (ii) allows direct genetic amplification from target pathogens due to a superior tolerance to well-known PCR inhibitors such as sputum (iii) demonstrates high specificity and sensitivity and (iv) results in rapid detection often within 30 min.

Huang, W. E: et al. Microbial Biotechnology (2020) 13(4):950-961; Yang, W. et al. medRxiv (2020) DOI: 10.110112020.03.02.20030130; Baek, Y H et al. Emerging Microbes & Infections (2020) 9(1):998-1007; Zhang, Y et al. medRxiv (2020) DOI: 10.1101/2020.02.26.20028373; Jiang, M. et al. Frontiers in Cellular and Infection Microbiology (2020) 10:1-16; and CN 111 321 249 A (2020) teach various SARS-CoV-2 RT-LAMP primer sets and uses thereof to detect SAR-CoV-2.

There is an urgent need for robust RT-LAMP assays for the rapid detection of SARS-CoV-2 including variants thereof. In addition, RT-LAMP assays allowing the differential diagnosis between infection and disease caused by SARS-CoV-2 and other highly human-pathogenic Beta-CoV group B/C (SARS-CoV, MERS-CoV) would be beneficial.

### SUMMARY

**The invention is as defined in the set of claims.**

The inventors have developed RT-LAMP primer sets targeting the N and RdRp genes which demonstrated a high analytical sensitivity with a limit of detection of 10 genome copies per reaction and a time-to result of less than 15 minutes. The amplification speed was increased or the limit-of-detection lower as compared to published primer sets (Lamb et al., PLOS ONE, 2020, June 12, 15:e0234682, doi: 10.1371; Yu et al., Clin. Chem., 2020 April 21, hvaa102.doi: 10.1093). Moreover, the optimized RT-LAMP primer sets demonstrated superior clinical sensitivity (100%) as compared to published primer set (50 %). The superior effects of the optimized RT-LAMP primers were observed in real-time monoplex assay and end-point dualplex QUASR assay. This is surprising since the N gene which is a usual target of molecular diagnosis because of the abundance of its mRNA was previously reported as showing a poor sensitivity compared to other SARS-CoV-2 target genes such as Nsp3 (Park et al., The Journal of Molecular Diagnostics, 22, 6, June 2020). In addition, the combination of a SARS-CoV-2 specific RT-LAMP primer set (targeting the N gene) and a Beta-CoV group B/C specific RT-LAMP primer set (targeting the RdRp gene) provides a differential diagnosis between SARS-CoV-2 infection and disease and infection and disease caused by other Betacoronavirus group B/C such as SARS-CoV and MERS-CoV. The RT-LAMP primer sets designed by the inventors allow the detection of SARS-CoV-2, including variants of concern (VOC).

The disclosure which is not part of the invention relates to a set of oligonucleotide primers for amplification of Severe acute respiratory syndrome coronavirus 2 (SARS-CoV-2) RNA which comprises at least a forward primer and a reverse primer targeting SARS-CoV-2 Nucleocapsid (N) gene selected from the group consisting of:
- a forward primer which hybridizes to a sequence of at least 10 consecutive nucleotides, preferably at least 15 consecutive nucleotides from positions 486 to 505, 73 to 91, 171 to 189, 208 to 227, 450 to 467, 468 to 487, or 538 to 555 of said N gene complement sequence; and
- a reverse primer which hybridizes to a sequence of at least 10 consecutive nucleotides, preferably at least 15 consecutive nucleotides from positions 752 to 771, 307 to 328, 450 to 467, 450 to 467, 675 to 674, 739 to 759, or 762 to 779, respectively of said N gene sequence; and the indicated positions being determined by alignment with SEQ ID NO: 1.

According to the invention, the first set of primers is for isothermal nucleic acid amplification of SARS-CoV-2 RNA and targets SARS-CoV-2 Nucleocapsid (N) gene of SEQ ID NO:1 and is a set E comprising primers consisting of sequences SEQ ID NO: 47 to 52. The disclosure which is not part of the invention also relates to a set of primers for isothermal nucleic acid amplification of SARS-CoV-2 RNA selected from the group of :
- a set A of primers comprising the sequences SEQ ID NO: 3 to 8 or variants thereof;
- a set B of primers comprising the sequences SEQ ID NO: 14 to 19 or variants thereof;
- a set C of primers comprising primers comprising the sequences SEQ ID NO: 25 to 30 or variants thereof;
- a set D of primers comprising the sequences SEQ ID NO: 36 to 41 or variants thereof;
- a set F of primers comprising the sequences SEQ ID NO: 58 to 63 or variants thereof;
- a set G of primers comprising the sequences SEQ ID NO: 69 to 74 or variants thereof;
- a set H of primers comprising the sequences SEQ ID NO: 80 to 85 or variants thereof;
- a set I of primers comprising the sequences SEQ ID NO: 91 to 96 or variants thereof;
- a set J of primers comprising the sequences SEQ ID NO: 102 to 107 or variants thereof; and
- a set K of primers comprising the sequences SEQ ID NO: 113 to 118 or variants thereof, wherein the variants hybridize to said N gene sequence or complement thereof.

The first set of primers according to an aspect of the present disclosure, is specific for SARS-CoV-2.

Another aspect of the disclosure which is not part of the invention except for the second set as defined in claim 7 relates to a second set of oligonucleotide primers for amplification of Severe acute respiratory syndrome coronavirus 2 (SARS-CoV-2) RNA, which amplifies a target sequence of at least 100 nucleotides of SARS-CoV-2 RNA-dependent RNA polymerase (RdRp) gene situated between any one of positions 1819 to 1838 and any one of positions 2057 to 2076; any one of positions 1763 to 1782 and any one of positions 2057 to 2076; any one of positions 1819 to 1838 and any one of positions 2114 to 2133; and any one of positions 1880 to 1899 and any one of positions 2161 to 2180 of said RdRp gene sequence; and the indicated positions being determined by alignment with SEQ ID NO: 2.

According to the disclosure which is not part of the invention except for the second set as defined in claim 7, the second set of oligonucleotide primers, comprises at least a forward and a reverse primer targeting SARS-CoV-2 RNA-dependent RNA polymerase (RdRp) gene selected from the group consisting of:
- a forward primer which hybridizes to a sequence of at least 10 consecutive nucleotides, preferably at least 15 consecutive nucleotides from positions 1838 to 1858, 1782 to 1799, 1838 to 1858, or 1899 to 1916 of said RdRp gene complement sequence; and
- a reverse primer which hybridizes to a sequence of at least 10 consecutive nucleotides, preferably at least 15 consecutive nucleotides from positions 2040 to 2057, 2040 to 2057, 2096 to 2114, or 2142 to 2161, respectively of said RdRp gene sequence; and the indicated positions being determined by alignment with SEQ ID NO: 2.

According to the disclosure which is not part of the invention except for the second set as defined in claim 7, the second set of primers is for isothermal nucleic acid amplification of SARS-CoV-2 RNA; preferably selected from the group consisting of:
- a set T of primers comprising the sequences SEQ ID NO: 179 to 184 or variants thereof;
- a set L of primers comprising the sequences SEQ ID NO: 124 to 129 or variants thereof;
- a set M of primers comprising the sequences SEQ ID NO: 135 to 140 or variants thereof;
- a set N of primers comprising the sequences SEQ ID NO: 146 to 151 or variants thereof;
- a set O of primers comprising the sequences SEQ ID NO: 157 to 162 or variants thereof;
- a set P of primers comprising the sequences SEQ ID NO: 168 to 173 or variants thereof;
- a set U of primers comprising the sequences SEQ ID NO: 190 to 195 or variants thereof; and
- a set V of primers comprising the sequences SEQ ID NO: 201 to 206 or variants thereof, wherein the variants hybridize to said RdRp gene sequence or complement thereof.

The second set of primers according to an aspect of the present disclosure, is specific for Betacoronavirus group B/C.

The first or second set of oligonucleotide primers according to an aspect of the present disclosure, comprise at least one labelled oligonucleotide primer; preferably labelled at the 5' end; most preferably a fluorescent oligonucleotide primer. According to the disclosure, the fluorescent oligonucleotide primer is a Forward or Backward inner primer for isothermal nucleic acid amplification of SARS-CoV-2 RNA; preferably a Backward inner primer for isothermal nucleic acid amplification of SARS-CoV-2 RNA.

Another aspect of the invention relates to a combination of oligonucleotide primers comprising at least a first set of primers specific for SARS-CoV-2 and a second set of primers specific for Betacoronavirus group B/C, wherein the first set of primers comprises the sequences SEQ ID NO: 47 to 52, which hybridize to said N gene sequence or complement thereof, and the second set of primers comprises the sequences SEQ ID NO: 179 to 184 which hybridize to the RNA-dependent RNA polymerase (RdRp) gene sequence of SEQ ID NO:2 or complement thereof.

According to the disclosure, the combination provides a differential diagnosis between infection and disease caused by SARS-CoV-2 and other Betacoronavirus group B/C such as SARS-CoV and MERS-CoV.

Another aspect of the invention relates to a kit for the detection of SARS-CoV-2 and/or Betacoronavirus group B/C RNA, comprising at least one set of oligonucleotide primers or a combination thereof according to the present invention. In some particular embodiments, the kit further comprises at least one short quenching probe complementary to a Forward or Backward inner primer for isothermal nucleic acid amplification of SARS-CoV-2 RNA; preferably a short complementary quenching probe comprising a sequence selected from the group consisting of: 5'-CCGCCATT-3', 5'-TGGCTGGC-3', 5'-ACAAGCTACA-3' (SEQ ID NO: 225) and 5'- ATACAACGTG -3' (SEQ ID NO: 226); preferably a short complementary quenching probe labelled at the 3' end with a black hole quencher.

Another aspect of the invention relates to the use of the kit according to the present invention, for the *in vitro* diagnosis of SARS-CoV-2 and/or Betacoronavirus group B/C infection and associated disease in a biological sample from a subject.

Another aspect of the invention relates to the use of the kit according to the present invention for the *in vitro* detection of a contamination with SARS-CoV-2 and/or Betacoronavirus group B/C in an environmental sample.

Another aspect of the invention relates to a method of detection of SARS-CoV-2and/ or Betacoronavirus group B/C RNA in a sample, comprising:
- subjecting said sample to an isothermal nucleic acid amplification reaction using a set of primers for amplifying a target sequence of said viral RNA, or a combination thereof, according to the presentinvention, and
- detecting the presence of an amplification product for said target sequence.

### DETAILED DESCRIPTION

The disclosure relates to sets of oligonucleotide primers targeting the N or RdRp genes for rapid and sensitive amplification, in particular isothermal nucleic acid amplification, and detection of Severe acute respiratory syndrome coronavirus 2 (SARS-CoV-2) nucleic acid. The oligonucleotide primer sets are useful alone or in combination for the specific diagnosis of SARS-CoV-2 and Betacoronavirus group B/C infections in a biological sample or detection of SARS-CoV-2 and Betacoronavirus group B/C contaminations in an environmental sample. The disclosure encompasses the derived Kit and method for the detection or diagnostics of SARS-CoV-2 and Betacoronavirus group B/C comprising at least a set of oligonucleotide primers or combination thereof according to the present disclosure. The sets of oligonucleotide primers according to the disclosure are useful for the detection and diagnosis of SARS-CoV-2 including variants of concern.

A first aspect of the disclosure relates to a first set of oligonucleotide primers for amplification of Severe acute respiratory syndrome coronavirus 2 (SARS-CoV-2) nucleic acid which comprises at least a forward primer and a reverse primer targeting SARS-CoV-2 Nucleocapsid (N) gene selected from the group consisting of:
- a forward primer which hybridizes to a sequence of at least 10 consecutive nucleotides, preferably at least 15 consecutive nucleotides from positions 486 to 505 of said N gene complement sequence and a reverse primer which hybridizes to a sequence of at least 10 consecutive nucleotides, preferably at least 15 consecutive nucleotides from positions 752 to 771 of said N gene sequence;
- a forward primer which hybridizes to a sequence of at least 10 consecutive nucleotides, preferably at least 15 consecutive nucleotides from positions 73 to 91 of said N gene complement sequence and a reverse primer which hybridizes to a sequence of at least 10 consecutive nucleotides, preferably at least 15 consecutive nucleotides from positions 307 to 328 of said N gene sequence;
- a forward primer which hybridizes to a sequence of at least 10 consecutive nucleotides, preferably at least 15 consecutive nucleotides from positions 171 to 189 of said N gene complement sequence and a reverse primer which hybridizes to a sequence of at least 10 consecutive nucleotides, preferably at least 15 consecutive nucleotides from positions 450 to 467 of said N gene sequence;
- a forward primer which hybridizes to a sequence of at least 10 consecutive nucleotides, preferably at least 15 consecutive nucleotides from positions 208 to 227 of said N gene complement sequence and a reverse primer which hybridizes to a sequence of at least 10 consecutive nucleotides, preferably at least 15 consecutive nucleotides from positions 450 to 467 of said N gene sequence;
- a forward primer which hybridizes to a sequence of at least 10 consecutive nucleotides, preferably at least 15 consecutive nucleotides from positions 450 to 467 of said N gene complement sequence and a reverse primer which hybridizes to a sequence of at least 10 consecutive nucleotides, preferably at least 15 consecutive nucleotides from positions 675 to 674 of said N gene sequence;
- a forward primer which hybridizes to a sequence of at least 10 consecutive nucleotides, preferably at least 15 consecutive nucleotides from positions 468 to 487 of said N gene complement sequence and a reverse primer which hybridizes to a sequence of at least 10 consecutive nucleotides, preferably at least 15 consecutive nucleotides from positions 739 to 759 of said N gene sequence; and
- a forward primer which hybridizes to a sequence of at least 10 consecutive nucleotides, preferably at least 15 consecutive nucleotides from positions 538 to 555 of said N gene sequence and a reverse primer which hybridizes to a sequence of at least 10 consecutive nucleotides, preferably at least 15 consecutive nucleotides from positions 762 to 779 of said N gene sequence; and the indicated positions being determined by alignment with SEQ ID NO: 1.

The Nucleocapsid (N) gene or ORF9 of SARS-CoV-2 (Gene ID: 43740575) corresponds to the nucleotide sequence from positions 28274 to 29533 of GenBank accession number NC_045512.2 accessed on March 30, 2020 or SEQ ID NO : 1 (1260 nt) which codes for the nucleocapsid phosphoprotein (GenPept accession number YP_009724397.2 accessed on March 30, 2020 ; 419 aa) comprising the Nucleocapsid (NP) protein from positions 14 to 368. SEQ ID NO: 1 is the sequence of the N gene from SARS-CoV-2 isolate Wuhan-Hu-1. One skilled in the art can easily determine the target sequence of the set of primers according to the invention in the N gene of any other SARS-CoV-2 isolate by alignment with SEQ ID NO: 1 using appropriate software available in the art such as BLAST, CLUSTALW and others.

As used herein "hybridize" refers to the ability to form a double-stranded hybrid molecule with SARS-CoV-2 nucleic acid (RNA, DNA equivalent or complement thereof) sufficient to produce a cDNA and to promote amplification of the cDNA in the conditions used for nucleic acid amplification such as those disclosed in the examples. The reverse primer is an anti-sense primer which hybridizes to SARS-CoV-2 RNA or DNA equivalent generated by amplification. The forward primer is a sense primer which hybridizes to SARS-CoV-2 cDNA (complementary DNA) or DNA copies thereof generated by amplification. All the sequences are provided in their 5' to 3" orientation. For example, the sequence from positions 486 to 505 of said N gene is 5'-TCAAGGAACAACATTGCCAA-3' (SEQ ID NO: 47); its complement is 5'-AGTTCCTTGTTGTAACGGTT-3' (SEQ ID NO: 223). The forward primer which hybridizes to at least 10 consecutive nucleotides, preferably at least 15 consecutive nucleotides from positions 486 to 505 of said N gene complement sequence is for example the forward primer 5'-TCAAGGAACAACATTGCCAA-3' (SEQ ID NO: 47). The sequence from positions 307 to 328 of said N gene sequence is 5'-CTGCTGAGGCTTCTAAGAAG-3' (SEQ ID NO: 224). The reverse primer which hybridizes to a sequence of at least 10 consecutive nucleotides, preferably at least 15 consecutive nucleotides from positions 307 to 328 of said N gene sequence is for example the primer 5'-CTTCTTAGAAGCCTCAGCAG-3' (SEQ ID NO: 48) which is the reverse-complement of SEQ ID NO: 224.

The set of primer according to the disclosure comprises a least a pair of primers (forward primer and reverse primer) and may comprise additional primers, in particular for isothermal nucleic amplification, the set of primers comprise 3 pairs of primers: External (F3 and B3); Internal (Forward inner primer or FIP(F1c+F2) and Backward inner primer or BIP (B1c+B2)); and Loop (Loop Forward or LoopF and Loop Backward (LoopB)).

Another aspect of the disclosure relates to a second set of oligonucleotide primers for amplification of Severe acute respiratory syndrome coronavirus 2 (SARS-CoV-2) nucleic acid which amplifies a target sequence of at least 100 nucleotides of SARS-CoV-2 RNA-dependent RNA polymerase (RdRp) gene situated between any one of positions 1819 to 1838 and any one of positions 2057 to 2076; any one of positions 1763 to 1782 and any one of positions 2057 to 2076; any one of positions 1819 to 1838 and any one of positions 2114 to 2133; and any one of positions 1880 to 1899 and any one of positions 2161 to 2180 of said RdRp gene sequence; and the indicated positions being determined by alignment with SEQ ID NO: 2.

The RNA-dependent RNA polymerase (RdRp) gene or nsp12 of SARS-CoV-2 corresponds to the sequence from positions 13442 to 13468 joined to positions 13468 to 16236 of the nucleotide sequence GenBank accession number NC_045512.2 accessed on March 30, 2020 or SEQ ID NO : 2 which codes for the RNA-dependent RNA polymerase (RdRp) protein (GenPept accession number YP_009725307.1 accessed on March 30, 2020 ; 932 aa). Positions 1782 to 1799; 2040 to 2057; 1838 to 1858; 2096 to 2114; 1899 to 1916 and 2142 to 2161 of SEQ ID NO: 2 correspond to positions 15223-15240, 15481-15498, 15279-15299, 15537-15555, 15340-15357, and 15583-15602 respectively of GenBank accession number NC_045512.2. The sequence SEQ ID NO: 2 is the sequence of the RdRp gene from SARS-CoV-2 isolate Wuhan-Hu-1. One skilled in the art can easily determine the target sequence of the set of primers according to the disclosure in the RdRp gene of any other SARS-CoV-2 isolate by alignment with SEQ ID NO: 2 or GenBank accession number NC_045512.2 using appropriate software available in the art such as BLAST, CLUSTALW and others.

The oligonucleotides according to the present disclosure which function as primers are capable of annealing specifically to a target sequence in the N or RdRp gene of SARS-CoV-2 nucleic acid and can be further extended in the presence of a nucleic acid polymerase to specifically amplify the target sequence. The target sequence refers to the particular nucleotide sequence of the target nucleic acid that is to be amplified and detected. The target sequence includes the sequences to which oligonucleotide primers hybridize during the nucleic acid amplification process. Where the target nucleic acid is originally single-stranded, the term target sequence will also refer to the sequence complementary to the target sequence as present in the target nucleic acid. Where the target nucleic acid is originally double-stranded the term target-sequence refers to both the sense (+) and antisense (-) strands. The target sequence consists of at least 50 consecutive nucleotides, preferably at least 100, 125 or 150 consecutive nucleotides from the target nucleic acid. Preferably, the target sequence consists of 100 to 350 consecutive nucleotides from the sequence of the target nucleic acid, more preferably 125 to 200 consecutive nucleotides. The target sequence is a unique sequence which is specific for SARS-CoV-2 or Betacoronavirus group B/C. Thus, the detection of the target sequence indicates the presence of SARS-CoV-2 or Betacoronavirus group B/C in the sample. Therefore, the detection of the target sequence in a biological sample is indicative of whether the individual is suffering from SARS-CoV-2 or Betacoronavirus group B/C virus infection and in particular from a disease caused by SARS-CoV-2 (Covid-19) or Betacoronavirus group B/C virus. The detection of the target sequence in an environmental sample is indicative of whether the environment is contaminated with SARS-CoV-2 or Betacoronavirus group B/C virus.

The target nucleic acid refers to a nucleic acid comprising a target sequence to be amplified. The target nucleic acid may include other sequences besides the target sequence, which may not be amplified. The viral nucleic acid or target nucleic acid may be genomic RNA (viral RNA or vRNA) or viral mRNA. In some preferred aspects of the disclosure, the target nucleic acid is viral RNA (*i.e.* SARS-CoV-2 or Betacoronavirus group B/C genomic RNA).

Oligonucleotide refers to a polymer of 5 to 100 nucleotides, generally from a lower limit of 15 to 20 nucleotides to an upper limit of 45 to 60 nucleotides, preferably from about 15 to about 45 nucleotides, wherein the polymer comprises ribonucleotides, deoxyribonucleotides, modified nucleotides or mixtures thereof and may further include modified internucleotide linkages and/or modified 5' and/or 3' termini. Oligonucleotides are usually synthesized using any of a variety of well-known enzymatic or chemical methods. The oligonucleotide primer according to the disclosure is substantially complementary to the target sequence. Substantially complementary means that the oligonucleotide is at least 80% identical, preferably at least 85%, 90%, 95% and 98% identical to the target sequence. The oligonucleotide may comprise additional sequences (not complementary to the target sequence) at its 5' end. In some aspects of the disclosure the oligonucleotide comprises a sequence of at least 5, preferably 10 to 15 consecutive nucleotides which is 100% identical to the target sequence. In some more preferred aspects of the disclosure, the oligonucleotide sequence is 100% identical to the target sequence. Optionally, at least one oligonucleotide of the pair includes a label (detectable moiety).

At least one oligonucleotide may also function as a probe and further includes a detectable label to detect a target nucleic acid or an amplicon thereof. The detectable label is a moiety that can be detected directly or indirectly by the production of a detectable signal (emission of energy) such as colorimetric, radioactive, fluorescent, luminescent (bioluminescent, chemiluminescent or electrochemoluminescent) signal. Directly detectable labels include radioisotopes and fluorophores. Indirectly detectable labels are detected by labeling with additional reagents that enable the detection. Indirectly detectable labels include, for example, chemiluminescent agents, enzymes that produce visible or colored reaction products, and a ligand-detectable ligand binding partner, where a ligand (hapten, antibody, antigen, biotin, avidin, epitope tag such as the FLAG epitope, enzyme tag such as horseradish peroxidase) may be detected by binding to a labelled ligand-specific binding partner. Preferred detectable labels include radioactive labels, fluorescent labels, chemiluminescent labels, bioluminescent labels, and epitope tags.

In some embodiments, one primer of the primer set is labelled, preferably with a fluorescent label such as 6-carboxy-fluorescein (6FAM), hexachloro-6-carboxy-fluorescein (HEX) and Texas red, TAMRA or Cy5, most preferably at the 5 'end. In some particular embodiments, the labelled primer is used in combination with a short complementary quenching probe. The short complementary quencher hybridizes to unincorporated primer upon cooling down at the end of RT-LAMP reaction, thereby quenching fluorescence of any unincorporated primer, according to QUASR technique (Quenching of Unincorporated Amplification Signal Reporters read). Preferably, the probe is labelled at its 3' end with a suitable non-fluorescent quencher such as black hole quencher 1 (BHQ1) or black hole quencher 2 (BHQ2). BHQ1 is used to quench green and yellow dyes (FAM, TET, HEX) and BHQ-2 is preferred for quenching orange or red dyes such as TAMRA, Texas Red and Cy5.

The oligonucleotides are designed based on SARS-CoV-2 and other Coronavirus group B/C including genomic sequences available in sequence databases using general principles for designing amplification primers. The design of the primers may also include specific principles for designing primers for use in specific isothermal amplification methods. Primer design may be performed using any of the various softwares.

As used herein, the expression "percentage of identity" between two nucleic acid sequences, means the percentage of identical nucleic acid, between the two sequences to be compared, obtained with the best alignment of said sequences, this percentage being purely statistical and the differences between these two sequences being randomly spread over the nucleic acid acids sequences. As used herein, "best alignment" or "optimal alignment", means the alignment for which the determined percentage of identity (see below) is the highest. Sequence comparison between two nucleic acids sequences is usually realized by comparing these sequences that have been previously aligned according to the best alignment; this comparison is realized on segments of comparison in order to identify and compare the local regions of similarity. The best sequences alignment to perform comparison can be realized, besides manually, by using the global homology algorithm developed by SMITH and WATERMAN (Ad. App. Math., vol.2, p:482, 1981), by using the local homology algorithm developed by NEDDLEMAN and WUNSCH (J. Mol. Biol, vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C, Nucleic Acids Research, vol. 32, p: 1792, 2004). To get the best local alignment, one can preferably use BLAST software. The identity percentage between two sequences of nucleic acids is determined by comparing these two sequences optimally aligned, the nucleic acids sequences being able to comprise additions or deletions in respect to the reference sequence in order to get the optimal alignment between these two sequences. The percentage of identity is calculated by determining the number of identical positions between these two sequences, and dividing this number by the total number of compared positions, and by multiplying the result obtained by 100 to get the percentage of identity between these two sequences.

As used herein, "Individual", "subject" or "patient" refers to a human or an animal. An animal includes a mammal and others.

The terms "a", "an", and "the" include plural referents, unless the context clearly indicates otherwise. For example "a primer" as used herein is understood to represent one or more primers. As such, the term "a" (or "an"), "one or more" or "at least one" can be used interchangeably herein.

As used herein, SARS-CoV-2 refers to any isolate, strain, lineage or variant of SARS-CoV-2. SARS-CoV-2 variant includes in particular variants of concern (VOC) such as alpha, beta, gamma, delta variants and other variants.

In some preferred aspects of the disclosure, the first set of oligonucleotide primers comprises a forward primer which hybridizes to a sequence of at least 10 consecutive nucleotides, preferably at least 15 consecutive nucleotides from positions 486 to 505 of said N gene complement sequence and a reverse primer which hybridizes to a sequence of at least 10 consecutive nucleotides, preferably at least 15 consecutive nucleotides from positions 752 to 771 of said N gene sequence.

In some particular aspects of the disclosure, the second set of oligonucleotide primers amplifies a target sequence of at least 100 nucleotides of SARS-CoV-2 RNA-dependent RNA polymerase (RdRp) gene situated between positions 1838 and 2057; 1782 and 2057; 1838 and 2114 ; and 1899 and 2161 of said RdRp gene sequence; and the indicated positions being determined by alignment with SEQ ID NO: 2.

In some particular aspects of the disclosure, the oligonucleotide primers of the second set are selected from the group consisting of the sequences of 5 to 100 nucleotides, preferably 15 to 45 nucleotides having 80% to 100% identity, preferably at least 85%, 90% 95% or 98% identity with any of the sequence from positions 1833 to 2062; 1777 to 2062; 1833 to 2119 ; and 1894 to 2166 of said RdRp gene sequence or the complement thereof; preferably any one of the sequence from positions 1838 to 2057; 1782 to 2057; 1838 to 2114; and 1899 to 2161 of said RdRp gene sequence or the complement thereof.

In some particular aspects of the disclosure, the second set of oligonucleotide primers comprises at least a forward primer and a reverse primer targeting SARS-CoV-2 RNA-dependent RNA polymerase (RdRp) gene selected from the group consisting of:
- a forward primer which hybridizes to a sequence of at least 10 consecutive nucleotides, preferably at least 15 consecutive nucleotides from positions 1838 to 1858 of said RdRp gene complement sequence and a reverse primer which hybridizes to a sequence of at least 10 consecutive nucleotides, preferably at least 15 consecutive nucleotides from positions 2040 to 2057 of said RdRp gene sequence;
- a forward primer which hybridizes to a sequence of at least 10 consecutive nucleotides, preferably at least 15 consecutive nucleotides from positions 1782 to 1799 of said RdRp gene complement sequence and a reverse which hybridizes to a sequence of at least 10 consecutive nucleotides, preferably at least 15 consecutive nucleotides from positions 2040 to 2057 of said RdRp gene sequence;
- a forward primer which hybridizes to a sequence of at least 10 consecutive nucleotides, preferably at least 15 consecutive nucleotides from positions 1838 to 1858 of said RdRp gene complement sequence and a reverse primer which hybridizes to a sequence of at least 10 consecutive nucleotides, preferably at least 15 consecutive nucleotides from positions 2096 to 2114 of said RdRp gene sequence; and
- a forward primer which hybridizes to a sequence of at least 10 consecutive nucleotides, preferably at least 15 consecutive nucleotides from positions 1899 to 1916 of said RdRp gene complement sequence and a reverse primer which hybridizes to a sequence of at least 10 consecutive nucleotides, preferably at least 15 consecutive nucleotides from positions 2142 to 2161 of said RdRp gene sequence; and the indicated positions being determined by alignment with SEQ ID NO: 2.

In some particular aspects of the invention, the first set of oligonucleotide primers is specific for SARS-CoV-2.

In various embodiments, SARS-CoV-2 is isolate Wuhan-Hu-1 or variant thereof such as alpha, beta, gamma, delta variants and other variants, in particular variants of concern. In some particular aspects of the disclosure, SARS-CoV-2 is selected from the group consisting of: isolate Wuhan-Hu-1, alpha, beta, gamma, and delta variants thereof and combinations thereof.

According to the invention , the second set of oligonucleotide primers of the invention is specific for Betacoronavirus group B/C. Betacoronavirus group B/C includes human pathogenic coronaviruses such as SARS-CoV and SARS-CoV-2 (Group B) and MERS-CoV (Group C). Betacoronavirus group B/C are different from other Betacoronavirus groups such as in particular Betacoronavirus group A which include OC43 which can cause the common cold and HKU1.

In some preferred aspects of the disclosure, the first set and/or the second set of oligonucleotide primers is for isothermal nucleic acid amplification of SARS-CoV-2 nucleic acid.

In some particular aspects of the disclosure, the first set of primers for isothermal nucleic acid amplification of SARS-CoV-2 nucleic acid is selected from the group consisting of:
- a set E of primers comprising the sequences SEQ ID NO: 47 to 52 or variants thereof;
- a set A of primers comprising the sequences SEQ ID NO: 3 to 8 or variants thereof;
- a set B of primers comprising the sequences SEQ ID NO: 14 to 19 or variants thereof;
- a set C of primers comprising primers comprising the sequences SEQ ID NO: 25 to 30 or variants thereof;
- a set D of primers comprising the sequences SEQ ID NO: 36 to 41 or variants thereof;
- a set F of primers comprising the sequences SEQ ID NO: 58 to 63 or variants thereof;
- a set G of primers comprising the sequences SEQ ID NO: 69 to 74 or variants thereof;
- a set H of primers comprising the sequences SEQ ID NO: 80 to 85 or variants thereof;
- a set I of primers comprising the sequences SEQ ID NO: 91 to 96 or variants thereof;
- a set J of primers comprising the sequences SEQ ID NO: 102 to 107 or variants thereof; and
- a set K of primers comprising the sequences SEQ ID NO: 113 to 118 or variants thereof.

The primer may comprise or consist of the above-mentioned sequence. The External and Loop primers for isothermal nucleic acid amplification consist usually of 15 to 25 nucleotides, whereas the Internal primers consist of 35 to 45 nucleotides. The variants or sequence variants refer to functional primers which hybridize to SARS-CoV-2 nucleic acid (RNA, DNA equivalent or complement thereof), e.g., have the ability to form a double-stranded hybrid molecule with SARS-CoV-2 nucleic acid (RNA, DNA equivalent or complement thereof) sufficient to produce a cDNA and to promote amplification of the cDNA in the conditions used for nucleic acid amplification such as those disclosed in the examples. For primers of up to 20 nucleotides, the sequence variants may comprise up to 5 nucleotide differences (1, 2, 3, 4 or 5 nucleotide differences) with the parent sequence; for primers of more than 20 nucleotides, the sequence variants may comprise up to 10 nucleotide differences (1, 2, 3, 4, 5, 6, 7, 8, 9, 10 nucleotide differences) with the parent sequence, for examples SEQ ID 47 to 52 for set E. One nucleotide difference refers to the deletion, insertion or substitution of one nucleotide. In some aspects of the disclosure, the sequence variant has at least 80% identity, preferably at least 85%, 90%, 95% and 98% identity with the parent sequence.

In some more preferred aspects of the disclosure, the first set of oligonucleotide primers for isothermal nucleic acid amplification of SARS-CoV-2 nucleic is selected from the group consisting of: a set A, B, C, E, F or G; preferably a set A, B, E, or F, or a set C, E, F, or G; more preferably a set C, E, or G; still more preferably a set E as defined above.

In some particular aspects of the disclosure, the second set of primers for isothermal nucleic acid amplification of SARS-CoV-2 nucleic acid is selected from the group consisting of:
- a set T of primers comprising the sequences SEQ ID NO: 179 to 184 or variants thereof;
- a set L of primers comprising the sequences SEQ ID NO: 124 to 129 or variants thereof;
- a set M of primers comprising the sequences SEQ ID NO: 135 to 140 or variants thereof;
- a set N of primers comprising the sequences SEQ ID NO: 146 to 151 or variants thereof;
- a set O of primers comprising the sequences SEQ ID NO: 157 to 162 or variants thereof;
- a set P of primers comprising the sequences SEQ ID NO: 168 to 173 or variants thereof;
- a set U of primers comprising the sequences SEQ ID NO: 190 to 195 or variants thereof; and
- a set V of primers comprising the sequences SEQ ID NO: 201 to 206 or variants thereof.

In some more preferred aspects of the disclosure, the second set of oligonucleotide primers for isothermal nucleic acid amplification of SARS-CoV-2 nucleic acid is selected from the group consisting of: a set L, M, T, U or V; or a set N, O, P or T; preferably a set T, U or V; or a set N, P or T; more preferably a set T as defined above.

In some preferred embodiments, the first or second set of oligonucleotide primers, in particular a set of oligonucleotide primer for isothermal nucleic acid amplification of SARS-CoV-2 nucleic acid, comprise at least one labelled oligonucleotide primer; preferably a fluorescent oligonucleotide primer. In some more preferred embodiment, the fluorescent primer is a Forward or Backward inner primer (FIP or BIP); most preferably a Backward inner primer. In some other more preferred embodiments, the fluorescent primer is used in combination with a short complementary quenching probe. The primer is most preferably labelled at the 5' end and the short complementary quenching probe labelled at the 3' end with a suitable quencher such as BHQ-1 or BHQ-2.

In some preferred aspects of the disclosure, the first set of oligonucleotide primers is a set E of primers comprising the sequences SEQ ID NO: 47 to 52 or variants thereof, wherein the BIP primer (SEQ ID NO: 50 or variant thereof) or FIP primer (SEQ ID NO: 49 or variant thereof) comprises a fluorescent label, preferably at the 5' end. In particular aspects of the disclosure, the fluorescent BIP or FIP primer is used in combination with a short complementary quenching probe, preferably comprising the sequence 5'-CCGCCATT-3' and 5'-TGGCTGGC-3', respectively for the BIP (SEQ ID NO: 50 or variant thereof) and FIP primer (SEQ ID NO: 49 or variant thereof). The quenching probe is preferably labelled at the 3'end with a suitable quencher such as BHQ1 or BHQ2. In some preferred aspects of the disclosure, the BIP primer (SEQ ID NO: 50 or variant thereof) comprises a fluorescent label, preferably at the 5' end. In some more preferred aspects of the disclosure, the BIP primer (SEQ ID NO: 50 or variant thereof) is labelled at the 5'end with Texas red and used in combination with a quenching probe comprising the sequence 5'-CCGCCATT-3' labelled at the 3' end with BHQ2.

In some preferred aspects of the disclosure, the second set of oligonucleotide primers is a set T of primers comprising the sequences SEQ ID NO: 179 to 184 or variants thereof, wherein the BIP primer (SEQ ID NO: 182 or variant thereof) or FIP primer (SEQ ID NO: 181 or variant thereof) comprises a fluorescent label, preferably at the 5' end. In particular aspects of the disclosure, the fluorescent BIP or FIP primer is used in combination with a short complementary quenching probe, preferably comprising the sequence 5'- ACAAGCTACA - 3' (SEQ ID NO: 225) and 5'- ATACAACGTG -3'(SEQ ID NO: 226), respectively for the BIP (SEQ ID NO: 182 or variant thereof) and FIP primer (SEQ ID NO: 181 or variant thereof). The quenching probe is preferably labelled at the 3'end with a suitable quencher such as BHQ1 or BHQ2. In some preferred aspects of the disclosure, the BIP primer (SEQ ID NO: 182 or variant thereof) comprises a fluorescent label, preferably at the 5' end. In some more preferred aspects of the disclosure, the BIP primer (SEQ ID NO: 182 or variant thereof) is labelled at the 5'end with FAM and used in combination with a quenching probe comprising the sequence 5'- ACAAGCTACA -3' (SEQ ID NO: 225) labelled at the 3' end with BHQ1.

Another aspect of the invention relates to a combination of oligonucleotide primers comprising at least a first set and a second set of oligonucleotide primers according to the present invention. In some aspects of the disclosure, the combination comprises a first set of oligonucleotide primers which is specific for SARS-CoV-2 and a second set of oligonucleotide primers which is specific for Betacoronavirus group B/C. According to the invention, , the combination comprises the set E and the set T. The combination of primer sets allows the differential diagnosis between infection and disease caused by SARS-CoV-2 and other Betacoronavirus group B/C such as SARS-CoV and MERS-CoV.

Another aspect of the present disclosure relates to a kit for the detection of SARS-CoV-2 and/or Betacoronavirus group B/C nucleic acid, comprising at least one pair or set of oligonucleotide primers for amplifying a target sequence of a SARS-CoV-2 nucleic acid according to the present disclosure. Preferably, at least one oligonucleotide of the set or pair comprises a detectable label. Preferably, the kit further comprises at least one short quenching probe complementary to a Forward or Backward inner primer for isothermal nucleic acid amplification of SARS-CoV-2 RNA according to the present disclosure, most preferably the short complementary quenching probe is labelled at the 3' end with a black hole quencher.

The kit optionally comprises reagents for the isothermal amplification of the target sequence and/or the detection of the amplification product. Reagents available for this purpose are well-known in the art and include the DNA polymerases and RT enzymes described above, buffers for the enzymes, detergents, enhancing agents, nucleic acid binding dyes and probes, preferably labelled probes. In some preferred aspects of the kit of the disclosure, the primers, and optional reagents are in lyophilised form to allow ambient storage. The components of the kits are packaged together into any of the various containers suitable for nucleic acid amplification such as plates, slides, wells, dishes, beads, particles, cups, strands, chips, strips and others. The kit optionally includes instructions for performing at least one specific aspect of the method of the disclosure. In some advantageous aspects of the disclosure, the kit comprises micro-well plates or microtubes, preferably in a dried format, *i.e.,* wherein the wells of the plates or microtubes comprise a dried composition containing at least the primers, and preferably further comprising all the reagents for the isothermal amplification of the target sequence and/or the detection of the amplification product amplification. In some other advantageous aspects of the disclosure, the primers and optional reagents are included into any of the devices available for nucleic acid amplification including devices further integrating nucleic acid extraction and/or amplification product detection capacities such as microfluidic devices or other devices. Examples of preferred microfluidic device include the COVIDISC developed by Professor Patrick Tabeling in the Laboratory Chimie Biologie Innovation at ESPCI Paris-PSL.

In some aspects of the disclosure, the kit is for the diagnosis of SARS-CoV-2 and/or Betacoronavirus group B/C infection or associated diseases such as Covid-19. In some other aspects of the disclosure, the kit is for the detection of a contamination with SARS-CoV-2 and/or Betacoronavirus group B/C in an environmental sample.

In some preferred aspects of the disclosure, the kit is for the detection of SARS-CoV-2 and/or Betacoronavirus group B/C by isothermal nucleic acid amplification, comprising at least a set of oligonucleotide primers for isothermal nucleic acid amplification of SARS-CoV-2 nucleic acid or a combination thereof according to the present disclosure. In some particular aspects of the disclosure, at least one oligonucleotide of the set comprises a detectable label. In some particular aspects of the disclosure, the kit comprises a first set of oligonucleotide primers which is specific for SARS-CoV-2 and a second set of oligonucleotide primers which is specific for Betacoronavirus group B/C. In some preferred aspects of the disclosure, the combination comprises the set E and the set T. In some particular aspects of the disclosure, at least one oligonucleotide of the set comprises a detectable label.

In some aspects of the disclosure, the kit further comprises at least one oligonucleotide probe or primer for the detection of another agent, in particular another pathogen, for example a human or animal pathogen. In some preferred aspects of the disclosure, the pathogen is a human pathogen. In some other aspects of the disclosure, the pathogen is a human pathogen.

Another aspect of the disclosure relates to the use of a set of oligonucleotide primers, combination or kit according to the present disclosure for the *in vitro* detection of SARS-CoV-2 or Betacoronavirus group B/C nucleic acid in a sample. Betacoronavirus group B/C includes human pathogenic coronaviruses such as SARS-CoV and SARS-CoV-2 (Group B) and MERS-CoV (Group C). Betacoronavirus group B/C are different from other Betacoronavirus groups such as in particular Betacoronavirus group A which include OC43 which can cause the common cold and HKU1.

According to the disclosure, SARS-CoV-2 may be detected using a set of oligonucleotide primers specific for SARS-CoV-2 or a combination of a set of oligonucleotide primers specific for SARS-CoV-2 and a set of oligonucleotide primers specific for Betacoronavirus group B/C. According to the disclosure, Betacoronavirus group B/C is detected using a set of oligonucleotide primers specific for Betacoronavirus group B/C. The combination of primer sets allows the differential diagnosis between SARS-CoV-2 infection and disease and infection and disease caused by other Betacoronavirus group B/C such as SARS-CoV and MERS-CoV.

In some aspects of the disclosure, the set of oligonucleotide primers, combination or kit according to the present disclosure are used for the diagnosis of SARS-CoV-2 and/or Betacoronavirus group B/C infection and associated disease such as Covid-19 in a biological sample from a subject.

In some aspects of the disclosure, the subject is a human. In some other aspects of the disclosure, the subject is an animal, including companion animals, livestock, and wild or zoo animals. Companion animals includes cat, dog and others.

In some other aspects of the disclosure, the set of oligonucleotide primers, combination or kit according to the present disclosure is used for the detection of a contamination with SARS-CoV-2 and/or Betacoronavirus group B/C in an environmental sample.

The detection is performed on any sample suspected of containing SARS-CoV-2 and/or Betacoronavirus group B/C. The sample includes any specimen that may contain SARS-CoV-2 or Betacoronavirus group B/C or components thereof such as nucleic acids or fragments of nucleic acids.

Samples include biological samples which refer to any material derived from living or dead individual (human or animal) that may contain SARS-CoV-2 or Betacoronavirus group B/C or target nucleic acid derived therefrom, including any tissue, body fluid or stool. Non-limiting examples of body fluids include whole-blood, serum, plasma, urine, cerebral spinal fluid (CSF), and mucosal secretions, such as with no limitations oral and respiratory tract secretions (sputa, saliva and the like). Samples include swabs such as oral or nasopharyngeal (NP) swabs, aspirate, wash or lavage. Samples for diagnostic tests for SARS-CoV-2 can be taken from the upper (nasopharyngeal/oropharyngeal swabs, nasal aspirate, nasal wash or saliva) or lower respiratory tract (sputum or tracheal aspirate or bronchoalveolar lavage (BAL). In some particular aspects of the disclosure, the biological sample is a clinical sample (*i.e.,* a sample from living or dead individual (human or animal) suspected of having SARS-CoV-2 or Betacoronavirus group B/C, preferably a body fluid sample, more preferably oral or respiratory tract secretions.

Samples also include environmental samples that may contain SARS-CoV-2 or Betacoronavirus group B/C such as air, water, soil, food, beverages, feed, water (e.g., fresh water, salt water, waste water, and drinking water), sewage, sludge, environmental surfaces and others. The environmental surface sample is for example a surface swab or swipe. Air sample may be collected using an air sampler such as Coriolis Micro (Bertin Instruments). In some particular aspects of the disclosure, the environmental sample is air or environmental surface.

Samples include direct samples (without nucleic isolation) and processed samples that have been treated to disrupt tissue or cell structure, thus releasing intracellular components into a solution which may further contain reagents (buffers, salts, detergents, enzymes and the like) which are used to prepare, using standard methods, a biological sample for analysis. In particular, processed samples include samples that have been treated by standard methods used for the isolation of nucleic acids from biological samples. In some aspects of the disclosure, the sample is a direct sample. In some other aspects of the disclosure, the sample has been treated to isolate nucleic acids.

Another aspect of the disclosure relates to a method of detection of SARS-CoV-2 and/or Betacoronavirus group B/C nucleic acid in a sample, comprising:
a) subjecting said sample to a nucleic acid amplification reaction using at least one pair or set of oligonucleotide primers or combination thereof for amplifying a target sequence of said viral nucleic acid according to the present disclosure, and
b) detecting the presence or absence of an amplification product for said target sequence.

In step a), of the method of the disclosure, the oligonucleotide primers which are contacted with the sample suspected of containing a human pathogenic coronavirus (SARS-CoV-2 or Betacoronavirus group B/C) are capable of hybridizing with the target sequence present in any SARS-CoV-2 or Betacoronavirus group B/C target nucleic acid present in said sample. The SARS-CoV-2 or Betacoronavirus group B/C target nucleic acid present in the sample is used as a template for generating an amplification product (amplicon). The amplification product includes monomers and concatemers of the target sequence. The amplification product (amplicon) may be detected by using a DNA binding dye or labelled primer, or hybridization assay using a labelled oligonucleotide probe. In step b) of the method of the disclosure, the detection of the presence or absence of the amplification product determines the presence or absence of SARS-CoV-2 or Betacoronavirus group B/C virus in the sample, and thereby whether or not the invidual is infected or the environment contaminated with SARS-CoV-2 or Betacoronavirus group B/C.

In some preferred aspects of the method of the disclosure, the nucleic acid amplification reaction (step a)) is an isothermal nucleic acid amplification reaction performed with at least one set of oligonucleotide primers for isothermal nucleic acid amplification according to the present disclosure.

In some preferred aspects of the disclosure, the method is a multiplex assay wherein several nucleic acid amplification reactions (step a), preferably isothermal nucleic acid amplification reactions, are performed simultaneously with at least two different sets of oligonucleotide primers for isothermal nucleic acid amplification according to the present disclosure. The nucleic acid amplification reactions are preferably performed with a set of oligonucleotide primer targeting the N gene and a set of oligonucleotide primer targeting the RdRp gene according to the present disclosure.

According to the method of the disclosure, the target sequence is amplified by isothermal nucleic acid amplification reaction using any of the various natural or engineered enzymes available for this purpose. These include in particular DNA polymerases having strong strand- displacement activity in isothermal conditions, thereby obviating the need for thermal cycling. Such polymerases are well-known in the art and include DNA polymerase long fragment (LF) of thermophilic bacteria such as *Bacillus stearothermophilus* (Bst), *Bacillus Smithii* (Bsm), *Geobacillus sp.* M (GspM) and *Thermodesulfatator indicus* (Tin), engineered variants therefrom as well as Taq DNA polymerase variants. Non-limiting examples of polymerase which can be used to perform the method of the disclosure include Bst LF DNA polymerase, GspM LF DNA polymerase, GspSSD LF DNA polymerase, Tin exo-LF DNA polymerase and SD DNA polymerase which are usually used at 50-75°C, more generally at 55 to 65°C.

In a particular aspect of the disclosure, the isothermal nucleic acid amplification reaction is performed at 65°C. In another particular aspect of the disclosure, the isothermal nucleic acid amplification reaction is performed using GspSSD LF DNA polymerase, preferably at 65°C.

According to another particular aspect of the disclosure, the isothermal amplification is chosen from strand-displacement amplification (SDA), Rolling-circle amplification (RCA), Cross-priming amplification (CPA), nucleic acid sequence-based amplification (NASBA), Recombinase Polymerase Amplification (RPA) and Loop-mediated amplification (LAMP). The principle of LAMP method is disclosed in Notomi et al., Nucleic Acids Res., 2000 Jun 15; 28(12):e63.

In some aspects of the disclosure, the amplification product of the isothermal nucleic acid amplification reaction is selected from the group consisting of the sequences SEQ ID NO: 13, 24, 35, 46, 57, 68, 79, 90, 101, 112, 123, 134, 145, 156, 167, 178, 189, 200 and 211; preferably 57 or 189.

Since the viral nucleic acid is RNA, the method of the disclosure comprise a reverse transcription reaction prior to or concomitantly with the amplification reaction of the target sequence. The reverse transcription reaction is performed using any reverse transcriptase (RT). RT are well-known in the art and include for example Avian Myeloblastosis Virus (AMV) and Moloney Murine Leukemia virus (MMLV) RT. The Reverse transcription and DNA amplification can be performed in the same reaction mixture comprising the DNA polymerase and RT enzymes. In addition, the disclosure can also use a DNA polymerase having both strong displacement activity and RT activity such as Pyrophage 3173 DNA polymerase.

The amplification product is detected using any of the various methods available for this purpose which are well-known in the art. For example, the detection method is fluorescence, bioluminescence, colorimetry, turbidimetry, immunoenzymatic or electrochemical detection. The detection may be semi-quantitative or quantitative. The detection may also be real-time detection, wherein the signal resulting from the presence of the amplification product is measured during the course of the nucleic acid amplification reaction to monitor the accumulation of specific amplification products or endpoint detection (after the amplification process). The disclosure allows for detection of the amplification product in the same vessel as amplification occurs. Fluorescence may use DNA intercaling dyes, fluorescent molecular beacon probes or a fluorescence metal indicator such as calcein. Colorimetry may use a colored indicator for alkaline metal ions, such as hydroxy naphthol blue (Goto et al., BioTechniques. 2009 Mar;46(3):167-72) or pH indicators (Tanner et al., BioTechniques, 2015 Feb;58(2):59-68). Turbidity is used when the amplification reaction, such as LAMP, produces large amounts of magnesium pyrophosphate (a white precipitate) and dsDNA, which allow visual inspection of results using a turbidimeter (Mori et al., Biochem. Biophys. Res. Commun., 2001 Nov 23; 289(1):150-4). Electrochemical detection may use a pH meter for direct measurement of released hydrogen ions during the amplification reaction, such as LAMP (Xie S et al., Chem. Commun., 2014 Oct 24; 50(100):15932-5), or integrated electrodes for measuring decreases in current resulting from increasing binding of electrochemically-active DNA-binding redox reporters, such as Methylene Blue, to amplification products (Xie et al., Biosens. Bioelectron. 2014 May 15; 55:324-9). Immunoassays include Enzyme-linked immunosorbent assays (ELISA) and lateral flow immunoassays based on the use of specific probes (Tsai S-M et al., J. Virol. Methods. 2012 Apr; 181(1):117-24; Ravan H and Yazdanparast R., Anal. Biochem., 2013 Aug 15; 439(2):102-8). Bioluminescence detection may be through measurement of bioluminescent output of the coupled conversion of inorganic pyrophosphate produced stoichiometrically during nucleic acid synthesis to ATP by the enzyme ATP sulfurylase (Gandelman et al., PLoS ONE., 2010 Nov 30; 5(11)).

In some aspects of the disclosure, the detection is real-time detection. In some other aspects of the disclosure, the detection is end-point detection. End-point detection is advantageously used in dualplex QUASR RT-LAMP assay, preferably using BIP or FIP primers labelled with different fluorophores and short complementary quenching probes according to the present disclosure.

According to another preferred aspect of the disclosure, the method comprises at least one non- SARS-CoV-2 internal control (IC) nucleic acid that is processed as the tested samples and amplified in the same assay reaction mixtures by using amplification oligonucleotide primers specific for a target sequence of the IC nucleic acid sequence. In some aspects of the disclosure, the IC target sequence is selected from the group consisting of: the sequence from positions 26 to 293, 227 to 498, or 387 to 621 of the CDS of human GAPDH gene (positions 189 to 1196 of GenBank NM_002046.5.2) and the sequence from positions 204 to 517, 584 to 869, or 908 to 1148 of the CDS of *Drosophila melanogaster* Sigma virus G gene (positions 4262 to 5947 of GenBank GQ375258).

According to another preferred aspect of the disclosure, the method comprises assaying several samples simultaneously. In some advantageous aspects of the disclosure, the method comprises assaying multiple samples simultaneously in a high-throughput process.

The method of the disclosure is useful for the diagnosis of SARS-CoV-2 and associated disease (Covid-19) in a biological sample from a subject. The method is also useful for the differential diagnosis between SARS-CoV-2 disease (Covid-19) and other Betacoronavirus group B/C diseases such as SARS-CoV and MERS-CoV diseases. The method is also useful for the detection of an environmental contamination with SARS-CoV-2. The method is also useful for the differential detection between a contamination with SARS-CoV-2 and a contamination with other Betacoronavirus group B/C such as SARS-CoV and MERS-CoV. In some aspects of the disclosure, the subject is a human subject. In other aspects of the disclosure, the subject is an animal including companion animals, livestock, and wild or zoo animals. Companion animals include cat, dog and others.

In some aspects of the disclosure, the method is a method of decontamination of an environment suspected to be contaminated with SARS-CoV-2 or Betacoronavirus group B/C, comprising the steps of:
a) subjecting an environmental sample suspected to be contaminated with SARS-CoV-2 or Betacoronavirus group B/C to a nucleic acid amplification reaction using at least one pair or set of oligonucleotide primers for amplifying a target sequence of said viral nucleic acid according to the present disclosure;
b) detecting the presence or absence of an amplification product for said target sequence; and
c) decontaminating the environment tested positive in step (b).

The decontamination may be performed by standard method that are well-known in the art using standard disinfectants that kill viruses including SARS-CoV-2 and/or Betacoronavirus group B/C.

In some aspects of the disclosure, the method is a method of controlling an infection with SARS-CoV-2 or Betacoronavirus group B/C, comprising the steps of:
a) subjecting biological sample(s) from subject(s) suspected to be contaminated with SARS-CoV-2 or Betacoronavirus group B/C to a nucleic acid amplification reaction using at least one pair or set of oligonucleotide primers for amplifying a target sequence of said viral nucleic acid according to the present disclosure;
b) detecting the presence or absence of an amplification product for said target sequence; and
c) isolating and/or treating subject(s) tested positive in step (b).

The treatment in step (c) may be a symptomatic and/or antiviral treatment using standard medicaments that are well-known in the art.

In some aspects of the disclosure, the method comprises repeating the nucleic acid detection according to steps (a) and (b) at least one time after the initiation of the treatment and/or isolation and continuing the isolation and/or treatment if the patient is tested positive or stopping the isolation and/or treatment if the patient is tested negative.

Another aspect of the disclosure is an oligonucleotide primer of the set of oligonucleotide primers according to the present disclosure. The oligonucleotide primer, in particular a labelled oligonucleotide primer is useful as a probe for the detection of SARS-CoV-2 nucleic acid including the specific detection of SARS-CoV-2 or Betacoronavirus group B/C nucleic acid.

In various the aspects of the disclosure, SARS-CoV-2 is isolate Wuhan-Hu-1 or variant thereof such as alpha, beta, gamma, delta variants and other variants, in particular variants of concern. In some particular aspects of the disclosure, SARS-CoV-2 is selected from the group consisting of: isolate Wuhan-Hu-1, alpha, beta, gamma, and delta variants thereof and combinations thereof.

The primer sets and derived kit, method, and use according to the disclosure allow the detection of SARSCoV-2 including variants of concern.

The practice of the present disclosure will employ, unless otherwise indicated, conventional techniques, which are within the skill of the art. Such techniques are explained fully in the literature.

The disclosure will now be exemplified with the following examples, which are not limitative, with reference to the attached drawings in which:

### FIGURE LEGENDS

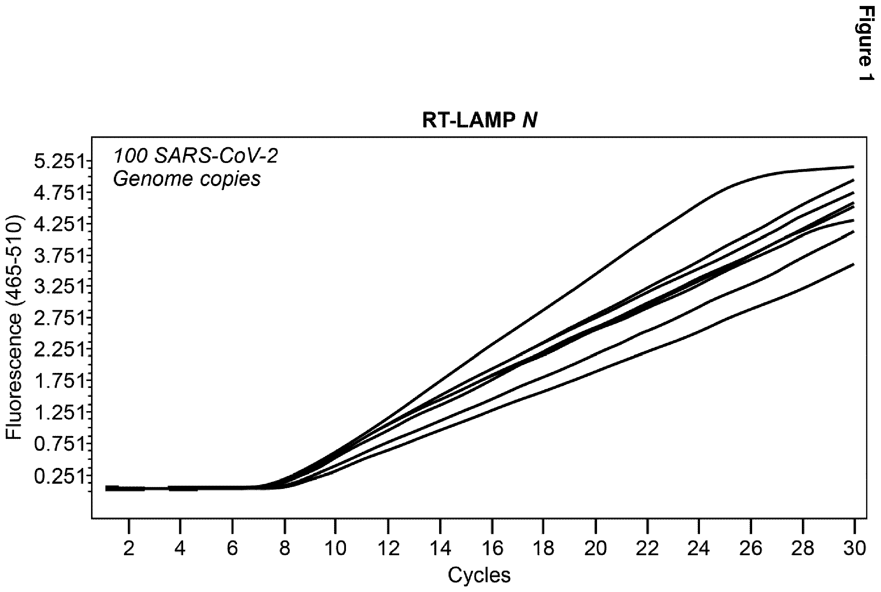

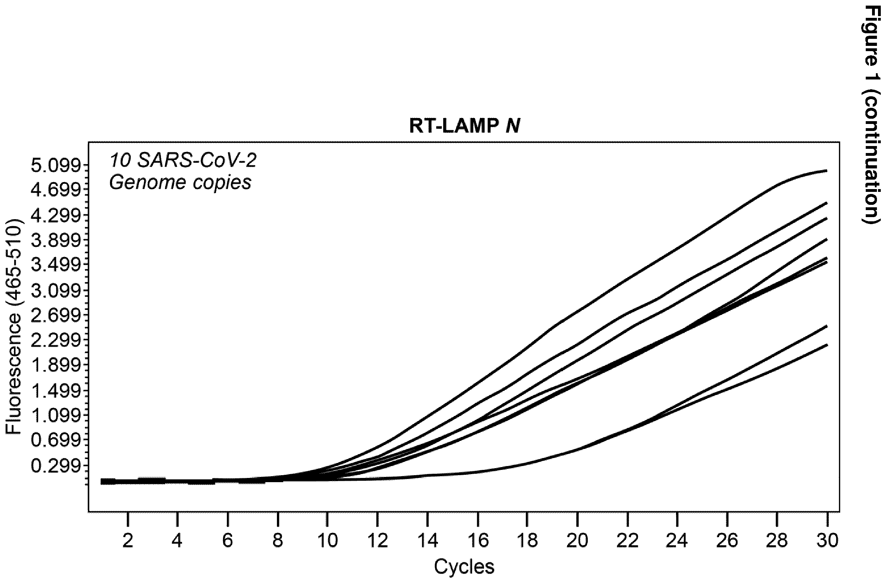

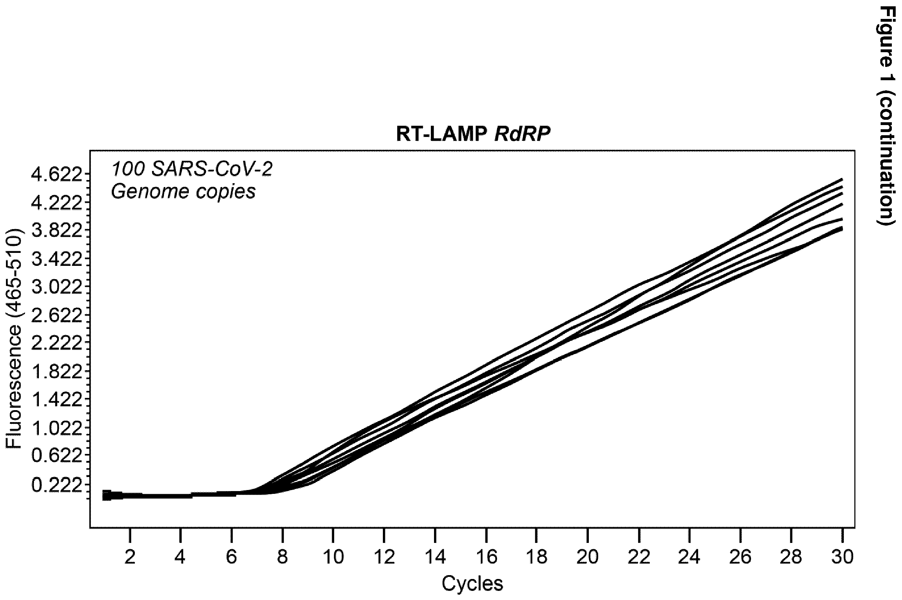

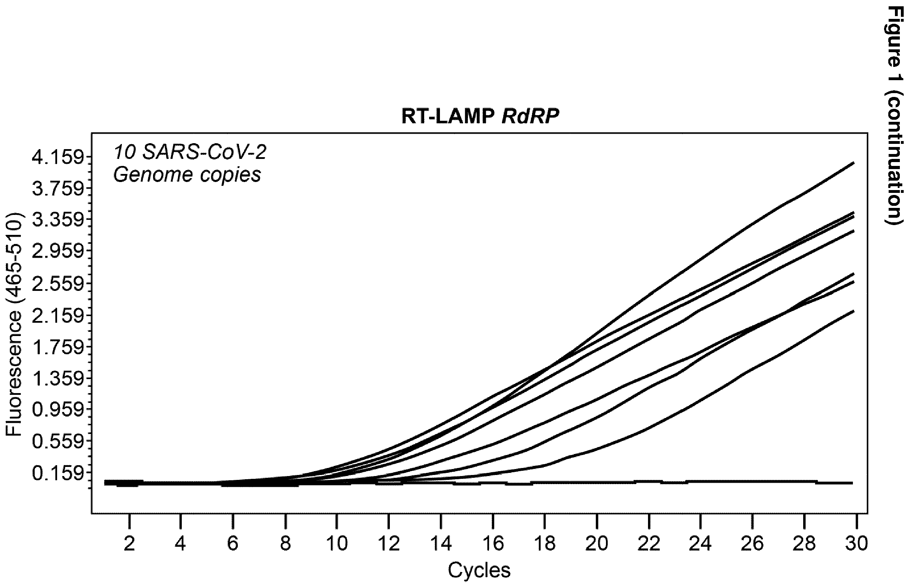

**Figure 1****. Amplification curves obtained for 10 and 100 genome copies of SARS-CoV-2 RNA template in a real-time SARS-CoV-2 RT-LAMP *N and RdRp* assay.**

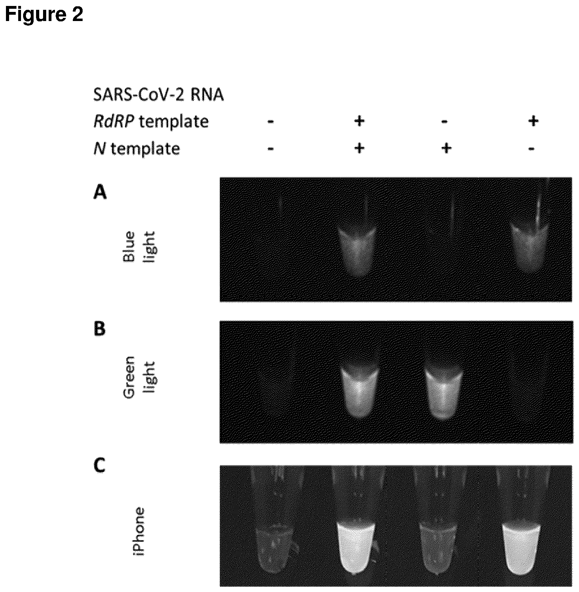

**Figure 2****. Visual detection of SARS-CoV-2 RNA by QUASR dualplex SARS-CoV-2 RT-LAMP assay (*N* and *RdRp*)*.*** A quantity of 10 copies of SARS-CoV-2 *RdRP* and/or *N* gene RNA template was used in each reaction where indicated by a plus sign. No template controls are indicated with a negative sign. When captured with a Z16 APO macroscope (Leica) equipped with FAM and Texas red cubes and an EM-CCD camera (Hamamatsu), SARS-CoV-2 *RdRP* positive templates appear bright when excited with blue light (A), and SARS-CoV-2 N positive templates appear bright when excited with green light (B). The image from a smartphone (iPhone 6) using an unfiltered blue LED excitation source and a plastic screen as an emission filter confirms multiplexed detection (C).

### EXAMPLES

### 1. Material and methods

### RT-LAMP primer design

Specific target gene regions were selected by multiple sequence alignments of all genome sequences available in the GISAID database for SARS-CoV-2 (as of March 27 2020) and primers specifically targeting selected gene regions were designed. Primer specificity was verified *in silico* by BLAST analysis. A total of 40 different primer set, each composed of 6 specific primers, were designed: 11 primer sets targeting the nucleoprotein (N) gene; 11 primer sets targeting the RNA-dependent RNA polymerase (*RdRp*) gene; and 7 primer sets targeting the *ORF3a*/*ORF4* region and 11 primer sets targeting the *ORF4*/*ORF5* region. In addition, two published RT-LAMP primer sets targeting the *ORF1a*/*b* were included in the performance evaluation study for comparison (Lamb et al., PLOS ONE, 2020, June 12, 15:e0234682, doi: 10.1371; Yu et al., Clin. Chem., 2020 April 21, hvaa102.doi: 10.1093).

### QUASR primer design

QUASR primers (*Quenching of unincorporated amplification signal reporters*) and their complementary quenching probes (quenchers) were designed with parameters adjusted for RT-LAMP reaction conditions: (i) fluorescently labeled primers were selected by avoiding primers that were likely to form stable hairpins, and (ii) the melting temperature of the fluorescent primer-quenching probe complex was designed to be significantly lower than 65°C. A total of 4 different QUASR primer sets were designed: 2 targeting the *RdRP* gene and 2 targeting the *N* gene. Each set was composed of 7 primers: a fluorescently-labeled internal primer, a complementary quenching probe, a second unmodified internal primer, two external primers and two loop primers. The primers targeting the *RdRp* gene are RdRp-FIP and RdRp-BIP (SEQ ID NO: 181 and 182; Table 3 and Table 4), the corresponding quenching probes are 5'-ATACAACGTG-3' (SEQ ID NO: 226) for RdRp-FIP and 5'- ACAAGCTACA -3' (SEQ ID NO: 225) for RdRp-BIP. The primers targeting the *N* gene are N-FIP and N-BIP (SEQ ID NO: 49 and 50; Table 3 and Table 4), the corresponding quenching probes are 5'-TGGCTGGC-3' for N-FIP and 5'-CCGCCATT-3' for N-BIP.

### Viral templates and sample preparation

Viral template controls included a positive control (internal quality control) consisting of an RNA extract, obtained from a pool of SARS-CoV-2 Coronavirus positive samples, diluted to a concentration of 20 genomic copies per µL (equivalent to a cycle threshold (Ct) of 35 in real-time RT-PCR. Respiratory specimens included nasopharyngeal swab eluates and saliva samples from suspect cases diagnosed negative or positive for SARS-CoV-2.

RNA was extracted from viral cultures or clinical specimens using the CE-IVD NucleoSpin^{®} Dx Virus kit (Macherey Nagel ref. 740895.50) or the CE-IVD BEC SARS-CoV-2 RT-LAMP kit for human samples, (ref. n° P05301). Saliva specimens were heat-treated for 5 minutes at 95°C in the presence of proteinase K according to the published, FDA EUA "SalivaDirect" protocol (Vogels et al., 2021) and used directly without further purification.

### Real-time and QUASR RT-LAMP assays

A generic real-time RT-LAMP protocol was developed for real-time detection of target SARS-CoV-2 genes. Briefly, a 25 µL RT-LAMP reaction was set up containing 15µL of ISO-004 master mix (Optigene), 1,3µL of the external, internal and loop primer mixture (final concentrations of 200, 1600 and 800 nM, respectively) and 5 µL template RNA. Primer sets were compared individually using the generic RT-LAMP protocol for optimal amplification and reaction time. Amplification was performed at a constant temperature of 65°C for 30 minutes. All reactions were conducted in a LightCycler 480 (Roche) or an ISO-8 (Axxin) instrument.

In addition, a generic QUASR RT-LAMP protocol was developed for end-point detection of target SARS-CoV-2 genes. Briefly, a 25 µL QUASR RT-LAMP reaction was set up containing 2,5 µL of iBuffer 5 (Optigene), 1,4 mM dNTP, 8 mM MgSO4, 0,8 M Betain, 8 units of GspSSD 2.0 polymerase (Optigene), 1,25 units of AMV-RT (Promega), 0,9 µL of the external, internal and loop primer mixture (final concentrations of 100, 800 and 400 nM, respectively), 1200 nM of complementary quencher and 5 µL template RNA. Primer sets were compared individually using the generic QUASR RT-LAMP protocol for optimal amplification and detection after 30 minutes of amplification at 65°C.

### Analytical sensitivity and specificity

To determine the analytical sensitivity of the *SARS-CoV-2* RT-LAMP assays, serial 10-fold dilutions of genomic *SARS-CoV-2* (extracted from a pool of CIBU107 and CIBU132 strains) were tested in parallel with the reference real-time RT-PCR developed by the National Reference Centre (NRC) for Respiratory Viruses in France (WHO Coronavirus disease COVID-19 technical guidance: Laboratory testing for 2019-nCoV in humans, available from https://www.who.int/docs/default-source/coronaviruse/whoinhouseassays.pdf? sfvrsn=de3a76aa_2) and the *SARS-CoV-2* RT-LAMP assays. Each dilution of template RNA was tested 8 times to determine intra-assay variability.

The recognition specificity of the RT-LAMP and QUASR RT-LAMP primers selected for the detection of Betacoronaviruses and SARS-CoV-2, including variants of concern (VOC) Alpha, Beta, Gamma and Delta, was determined *in silico* based on the analysis of sequences available in international databases. It was also experimentally controlled on extracts of human pathogenic RNA viruses commonly handled in the laboratory, and a panel of SARS-CoV-2 VOC strains:
- SARS-Coronavirus-2 Alpha VOC
- SARS-Coronavirus-2 Beta VOC
- SARS-Coronavirus-2 Gamma VOC
- Betacoronavirus group B/C: MERS-Coronavirus and SARS-Coronavirus
- Human Coronavirus OC43 (Betacoronavirus group A)
- Human Coronavirus 229E (Alphacoronavirus)
- Influenza A/H1N1
- Influenza A/H5N1
- Influenza A/H3N2
- Influenza B

### Clinical sensitivity and specificity

The specificity and sensitivity of the *SARS-CoV-2* RT-LAMP assays was determined on 45 respiratory specimen extracts from suspect cases diagnosed negative for SARS-CoV-2 and 62 respiratory specimen extracts from suspect cases diagnosed positive for SARS-CoV-2 were tested in the RT-LAMP assay.

### 2. Results

The *RdRp* gene encoding the RNA-dependent RNA polymerase and the *N* gene encoding the nucleoprotein were selected as optimal targets for the detection of SARS-CoV-2 virus. The RT-LAMP assays primer list is presented in **Table 1.**

Serial 10-fold dilutions of genomic SARS-CoV-2 (extracted from a pool of CIBU107 and CIBU132 strains) were prepared, aliquoted and tested 8 times using the various RT-LAMP master mixes to determine conditions for optimal reaction efficiency.

**Table 2: Optimization of RT-LAMP assay reaction master mix compositions**

| **RT-LAMP Primer Set ID** | **Limit of detection (genome copies per reaction)** | **Mean time to result (min)** |
|---|---|---|
| **A*(comparative)** | 10 | 25,41 |
| **B(comparative)** | 10 | 24,26 |
| **C(comparative)** | 100 | 7,45 |
| **D(comparative)** | 100 | 23,31 |
| **E** | **10** | **11,48** |
| **F(comparative)** | 10 | 9,91 |
| **G(comparative)** | 100 | 9,2 |
| **H(comparative)** | 100 | 14,28 |
| **I(comparative)** | 100 | 13,59 |
| **J(comparative)** | 100 | 21,35 |
| **K(comparative)** | 100 | 19,9 |
| **L**(comparative)** | 10 | 16, 26 |
| **M(comparative)** | 10 | 17,14 |
| **N(comparative)** | 100 | 10,14 |
| **O(comparative)** | 100 | 11,63 |
| **P(comparative)** | 100 | 11,45 |
| **T** | **10** | **11,21** |
| **U(comparative)** | 10 | 15,22 |
| **V(comparative)** | 10 | 16,48 |
| **AC***(comparative)** | 1000 | 25,00 |
| **Yu et al. 2020°** | 100 | 11,67 |
| **Lamb et al. 2020°** | 10 | 11,9 |

| | | |
|---|---|---|
| * N gene; ** RdRp; *** ORF3a/ORF4 °ORF1a/b | | |

**Table 3. SARS-CoV-2 RT-LAMP assay primer list**

| Target gene | Primer ID | Primer type | Primer sequence |
|---|---|---|---|
| *N* gene (set E) | N-F3 | External | TCAAGGAACAACATTGCCAA (SEQ ID NO : 47) |
| | N-B3 | External | CTTCTTAGAAGCCTCAGCAG (SEQ ID NO : 48) |
| | N-FIP | Internal | GCCAGCCATTCTAGCAGGAGGTAGTCGCAACAGTTC AAGA (SEQ ID NO : 49) |
| | N-BIP | Internal | AATGGCGGTGATGCTGCTTCTCAAGCTGGTTCAATC TG (SEQ ID NO : 50) |
| | N-LF | Loop | TGCTGCCTGGAGTTGAATT (SEQ ID NO: 51) |
| | N-L3 | Loop | TTGCTTTGCTGCTGCTTG (SEQ ID NO : 52) |
| *RdRP* gene (set T) | RdRP-F3 | External | CCTTATGGGTTGGGATTATCC (SEQ ID NO : 179) |
| | RdRP-B3 | External | TGTGGCATCTCCTGATGA (SEQ ID NO : 180) |
| | RdRP-FIP | Internal | CACGTTGTATGTTTGCGAGCAAGTGATAGAGCCATG CCTAAC (SEQ ID NO : 181) |
| | RdRP-BIP | Internal | TGTAGCTTGTCACACCGTTTCTCCACACATGACCATT TCACT ((SEQ ID NO : 182) |
| | RdRP-LF | Loop | AGTGAGGCCATAATTCTAAGCA (SEQ ID NO : 183) |
| | RdRP-L3 | Loop | AGCTAATGAGTGTGCTCAAGT (SEQ ID NO : 184) |

Among the tested RT-LAMP primer sets, primer sets E and T were identified as best performing primers as they demonstrated the lowest limit of detection with the fastest time-to-result, and were not associated with non-specific amplification due to self-priming artifacts **(Tables 2 & 3).**

While all QUASR RT-LAMP primer sets showed optimal amplification of target RNA, the 2 sets targeting the *RdRP* and *N* gene with a fluorescently-labeled backwards inner primer (BIP) showed a slightly stronger signal-to-noise ratio compared to fluorescently-labeled forward inner primer (FIP). These were therefore combined and used for all subsequent dualplex QUASR RT-LAMP assays **(Table 4).**

**Table 4. SARS-CoV-2 dualplex QUASR RT-LAMP assay primer list**

| Target genes | Primer ID | Primer type | Primer sequence (modification) |
|---|---|---|---|
| *N* and *RdRP* gene | N-F3 | External | TCAAGGAACAACATTGCCAA |
| | N-B3 | External | CTTCTTAGAAGCCTCAGCAG |
| | N-FIP | Internal | GCCAGCCATTCTAGCAGGAGGTAGTCGCAACAGTTCAAGA |
| | N-BIP-TxR | Labeled Internal | [5'TxR]AATGGCGGTGATGCTGCTTCTCAAGCTGGTTCAATCTG |
| | N-LF | Loop | TGCTGCCTGGAGTTGAATT |
| | N-L3 | Loop | TTGCTTTGCTGCTGCTTG |
| | N-Q-BHQ2 | Quencher | CCGCCATT [3'BHQ2] |
| | RdRP-F3 | External | CCTTATGGGTTGGGATTATCC |
| | RdRP-B3 | External | TGTGGCATCTCCTGATGA |
| | RdRP-FIP | Internal | CACGTTGTATGTTTGCGAGCAAGTGATAGAGCCATGCCTAAC |
| | RdRP-BIP-FAM | Labeled Internal | [5'FAM]TGTAGCTTGTCACACCGTTTCTCCACACATGACCATTTCACT |
| | RdRP-LF | Loop | AGTGAGGCCATAATTCTAAGCA |
| | RdRP-L3 | Loop | AGCTAATGAGTGTGCTCAAGT |
| | RdRP-Q-BHQ1 | Quencher | ACAAGCTACA [3'BHQ1] (SEQ ID NO: 225) |

Serial dilutions of *SARS-CoV-2* genomic RNA were used to determine the analytical sensitivity of the RT-LAMP and QUASR assays. The real-time *SARS-CoV-2* RT-LAMP assay demonstrated a limit of detection of 10 genome copies per reaction, with a time-to-result of less than 15 minutes **(Table 5).**

The end-point dualplex QUASR RT-LAMP assay demonstrated an equal limit of detection of 10 genome copies per reaction, after a reaction time of 30 minutes **(Table 5).**

**Table 5. Analytical sensitivity (limit of detection) of the real-time RT-LAMP assays and end-point QUASR dualplex RT-LAMP assays as compared to the gold-standard RT-PCR assay**

| Assay | Target gene | Primer set ID | Quantified genomic SARS-CoV-2RNA | | | | | |
|---|---|---|---|---|---|---|---|---|
| | | | 10 genome copies | | | 100 genome copies | | |
| | | | Mean Cp | SD Cp | Detected replicates (out of 8) | Mean Cp | SD Cp | Detected replicates (out of 8) |
| **Real-time RT-PCR** | | | | | | | | |
| | *RdRp* | IP2 (FAM) | 37.02 | 0.56 | 8 | 34.65 | 0.48 | 8 |
| | | IP4 (HEX) | 38.18 | 0.86 | 7 | 35.05 | 0.36 | 8 |

| **Real-time RT-LAMP** | | | | | | | | |
|---|---|---|---|---|---|---|---|---|
| | *N* | **E** | **11.48** | **2.66** | **8** | **7.95** | **0.38** | **8** |
| | *RdRP* | **T** | **11.21** | **2.47** | **7** | **7.59** | **0.71** | **8** |
| | *ORF1ab* | Yu *et* al., 2020 | 14.34 | - | 1 | 11.68 | 0.95 | 8 |
| | *ORF1ab* | Lamb *et* al., 2020 | 11.90 | 1.51 | 8 | 8.57 | 0.48 | 8 |

| **End-point dualplex QUASR RT-LAMP** | | | | | | | | |
|---|---|---|---|---|---|---|---|---|
| | *N* | E-BIP (TexasRed) | N/A | N/A | 8 | N/A | N/A | 8 |
| | *RdRp* | T-BIP (FAM) | N/A | N/A | 8 | N/A | N/A | 8 |

| | | | | | | | | |
|---|---|---|---|---|---|---|---|---|
| Cp, crossing point value, expressed in cydes(real-time RT-PCR) or minutes (real-time RT-LAMP) ; NiA, Not applicable | | | | | | | | |

Amplification curves obtained by RT-LAMP for 10 and 100 genome copies of *SARS-CoV-2* RNA template per reaction in a real time SARS-CoV-2 RT-LAMP N and RdRp assay are shown in **Figure 1****.** Visual detection of SARS-CoV-2 RNA by QUASR dualplex SARS-CoV-2 RT-LAMP assay (*N* and *RdRp*) is shown in **Figure 2****.**

The recognition specificity of real-time RT-LAMP primers selected for the detection of Betacoronaviruses and SARS-CoV-2, including variants of concern (VOC) Alpha, Beta, Gamma and Delta, was determined in silico based on the analysis of sequences available in international databases. It was also experimentally controlled on extracts of human pathogenic RNA viruses commonly handled in the laboratory, and a panel of SARS-CoV-2 VOC strains:
- SARS-Coronavirus-2 Alpha VOC
- SARS-Coronavirus-2 Beta VOC
- SARS-Coronavirus-2 Gamma VOC
- Betacoronavirus group B/C: MERS-Coronavirus and SARS-Coronavirus
- Coronavirus OC43 (Betacoronavirus group A)
- Coronavirus 229E (alphacoronavirus)
- Influenza A/H1N1
- Influenza A/H5N1
- Influenza A/H3N2
- Influenza B

Specificity was also evaluated on 45 respiratory specimen extracts from suspect cases diagnosed negative for SARS-CoV-2.

No cross-detection was found between the methods evaluated and the DNA/RNA viruses commonly handled in the laboratory or respiratory specimen extracts from suspect cases diagnosed negative for SARS-CoV-2. Therefore, no false-positive results were observed for the different tests. The specificity is above the laboratory's threshold for rapid tests (98%), **(Table 6).**

The sensitivity of the real-time RT-LAMP tests was evaluated on 62 respiratory specimen extracts from suspect cases diagnosed positive for SARS-CoV-2. The sensitivity is above the threshold retained by the laboratory for rapid tests (95%), **(Table 6).** The sensitivity of the dualplex QUASR RT-LAMP tests was evaluated on 20 respiratory specimens (saliva) from suspect cases diagnosed positive for SARS-CoV-2. The sensitivity is above the threshold retained by the laboratory for rapid tests (95%), **(Table 6).**

**Table 6: Clinical sensitivity and specificity**

| **RT-LAMP assay format** | **Assay target** | **Specimen** | **Results** | **Analytic Performances** |
|---|---|---|---|---|
| | | Nasopharyngeal swab extract | True positives 62/64 | |
| Real-time monoplex assay | βCoV group B/C | | False positives 0/51 | Specificity = 100 % |
| | | | True negatives 51/51 | Sensitivity = 97 % |
| | | | False negatives 2/64 | |
| Real-time monoplex assay | SARS-CoV-2 | Nasopharyngeal swab eluate extract | True positives 59/62 | |
| | | | False positives 0/53 | Specificity = 100 % |
| | | | True negatives 53/53 | Sensitivity = 95 % |
| | | | False negatives 3/62 | |
| End-point dualplex QUASR assay | βCoV group B/C | Saliva | True positives 20/20 | |
| | | | False positives 0/22 | Specificity = 100 % |
| | | | True negatives 22/22 | Sensitivity = 100 % |
| | | | False negatives 0/20 | |
| | SARS-CoV-2 | Saliva | True positives 19/20 | |
| | | | False positives 0/22 | Specificity = 100 % |
| | | | True negatives 22/22 | Sensitivity = 95 % |
| | | | False negatives 1/20 | |

A subset of 10 patient swab extracts, collected from 4 SARS-CoV-2 cases and 6 negative SARS-CoV-2 individuals were tested by RT-LAMP using primer subsets E, T and the primer set published by Lamb et al. While all primer sets were equally specific (no false positive results), primer sets E and T demonstrated superior clinical sensitivity (100%) as compared to the primer set from Lamb et al on the tested SARS-CoV-2 positive samples (50%) **(Table 7).**

**Table 7: Comparison of RT-LAMP assay performances on a panel of clinical samples**

| RT-LAMP Primer set ID | Number of positive samples detected | Number of negative samples detected |
|---|---|---|
| E | 4/4 | 6/6 |
| T | 4/4 | 6/6 |
| Lamb et al., 2020 | 2/4 | 6/6 |

In conclusion, the developed real-time and endpoint (QUASR) RT-LAMP assays demonstrated equivalent analytical sensitivity and a faster time-to-result (30 minutes as compared to 1 hour 45 minutes) as compared to the currently used reference real-time RT-PCR assays, making it highly suitable for rapid, reliable diagnostic applications. In addition the selected optimized RT-LAMP primer sets (E and T) showed either increased amplification speed or a lower limit-of-detection as compared to published primer sets. Moreover, the selected optimized RT-LAMP primer sets (E and T) demonstrated superior clinical sensitivity (100%) as compared to published primer set (50 %).

| **Table 1. RT-LAMP assays primer list** | | | | | | | | | |
|---|---|---|---|---|---|---|---|---|---|
| **Primer Set ID** 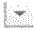 | **Target genome (accession nr)** 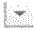 | **Target region** 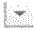 | **Position** 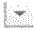 | **Name** 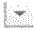 | **Sequence (5' to 3')** 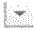 | **Primer position** 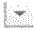 | **Primer length** 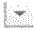 | | **SEQ ID NO:** 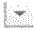 |
| **A** | SARS-CoV-2 (NC_045512) | N gene | 450 | F3 | TCCTGCTAACAATGCTGC | | 450 | 18 | 3 |
| | | | 694 | B3 | TCTCAAGCTGGTTCAATCTG | | 694 | 20 | 4 |
| | | | | FIP(F1c+F2) | AACGAGAAGAGGCTTGACTGCTCAAGGAACAACATTGCCA | | | 40 | 5 |
| | | | | BIP(Ble+B2) | CTCATCACGTAGTCGCAACAGTATTGCCAGCCATTCTAGC | | | 40 | 6 |
| | | | 524 | LoopF | CCTTCTGCGTAGAAGCCTT | | 524 | 19 | 7 |
| | | | 586 | LoopB | AATTCAACTCCAGGCAGCA | | 586 | 19 | 8 |
| | | | 486 | F2 | TCAAGGAACAACATTGCCA | | 486 | 19 | 9 |
| | | | 556 | F1c | AACGAGAAGAGGCTTGACTGC | | 556 | 21 | 10 |
| | | | 639 | B2 | ATTGCCAGCCATTCTAGC | | 639 | 18 | 11 |
| | | | 558 | B1c | CTCATCACGTAGTCGCAACAGT | | 558 | 22 | 12 |
| | | | | Product | TCAAGGAACAACATTGCCAAAAGGCTTCTACGCAGAAGGGAGCAGAG GCGGCAGTCAAGCCTCTTCTCGTTCCTCATCACGTAGTCGCAACAGTT CAAGAAATTCAACTCCAGGCAGCAGTAGGGGAACTTCTCCTGCTAGAA TGGCTGGCAAT | | | 154 | 13 |
| **B** | SARS-CoV-2 (NC_045512) | N gene | 486 | F3 | TCAAGGAAC AACATTGCCA | | 486 | 19 | 14 |
| | | | 771 | B3 | CTTCTTAGAAGCCTCAGCAG | | 771 | 20 | 15 |
| | | | | FIP(F1c+F2) | GCCAGCCATTCTAGCAGGAGCACGTAGTCGCAACAGTT | | | 38 | 16 |
| | | | | BIP(B1c+82) | AATGGCGGTGATGCTGCTTCTC AAGCTGGTTCAATCTG | | | 38 | 17 |
| | | | 604 | LoopF | TGCTGCCTGGAGTTGAATT | | 604 | 19 | 18 |
| | | | 656 | LoopB | TTGCTTTGCTGCTGCTTG | | 656 | 18 | 19 |
| | | | 563 | F2 | CACGTAGTCGCAACAGTT | | 563 | 18 | 20 |
| | | | 636 | F1c | GCCAGCCATTCTAGCAGGAG | | 636 | 20 | 21 |
| | | | 694 | B2 | TCTCAAGCTGGTTCAATCTG | | 694 | 20 | 22 |
| | | | 637 | B1c | AATGGCGGTGATGCTGCT | | 637 | 18 | 23 |
| | | | | Product | CACGTAGTCGCAACAGTTCAAGAAATTCAACTCCAGGCAGCAGTAGG GGAACTTCTCCTGCTAGAATGGCTGGCAATGGCGGTGATGCTGCTCTT GCTTTGCTGCTGCTTGACAGATTGAACCAGCTTGAGA | | | 132 | 24 |
| **C** | SARS-CoV-2 (NC_045512) | N gene | 486 | F3 | TCAAGGAACAACATTGCCA | | 486 | 19 | 25 |
| | | | 771 | B3 | CTTCTTAGAAGCCTCAGCAG | | 771 | 20 | 26 |
| | | | | FIP(F1c+F2) | GCCAGCCATTCTAGCAGGAGCGTAGTCGCAACAGTTCA | | | 38 | 27 |
| | | | | BIP(B1c+B2) | AATGGCGGTGATGCTGCTTCTCAAGCTGGTTCAATCTG | | | 38 | 28 |
| | | | 604 | LoopF | TGCTGCCTGGAGTTGAATT | | 604 | 19 | 29 |
| | | | 656 | LoopB | TTGCTTTGCTGCTGCTTG | | 656 | 18 | 30 |
| | | | 565 | F2 | CGTAGTCGCAACAGTTCA | | 565 | 18 | 31 |
| | | | 636 | F1c | GCCAGCCATTCTAGCAGGAG | | 636 | 20 | 32 |
| | | | 694 | B2 | TCTCAAGCTGGTTCAATCTG | | 694 | 20 | 33 |
| | | | 637 | B1c | AATGGCGGTGATGCTGCT | | 637 | 18 | 34 |
| | | | | Product | CGTAGTCGCAACAGTTCAAGAAATTCAACTCCAGGCAGCAGTAGGGG AACTTCTCCTGCTAGAATGGCTGGCAATGGCGGTGATGCTGCTCTTGC TTTGCTGCTGCTTGACAGATTGAACCAGCTTGAGA | | | 130 | 35 |
| **D** | SARS-CoV-2 (NC_045512) | N gene | 468 | F3 | AATCGTGCTACAACTTCCTC | | 468 | 20 | 36 |
| | | | 759 | B3 | CTCGCAGCAGATTTCTTAGT | | 759 | 21 | 37 |
| | | | | FIP(F1c+F2) | CCTACTGCTGGCTGGAGTTGAGTCAAGCCTCTTCTCGT | | | 38 | 38 |
| | | | | BIP(B1c+82) | CTCCTGCTAGAATGGCTGGCTCAATCTGTCAAGCAGCAG | | | 39 | 39 |
| | | | 575 | LoopF | TTGCG ACT ACGTGATGAGG | | 575 | 19 | 40 |
| | | | 643 | LoopB | GGTGATGCTGCTCTTGCT | | 643 | 18 | 41 |
| | | | 538 | F2 | AGTCAAGCCTCTTCTCGT | | 538 | 18 | 42 |
| | | | 609 | F1c | CCTACTGCTGCCTGGAGTTG | | 609 | 20 | 43 |
| | | | 682 | B2 | TCAATCTGTCAAGCAGCAG | | 682 | 19 | 44 |
| | | | 617 | B1c | CTCCTGCTAGAATGGCTGGC | | 617 | 20 | 45 |
| | | | | Product | AGTCAAGCCTCTTCTCGTTCCTCATCACGTAGTCGCAACAGTTCAAGA AATTCAACTCCAGGCAGCAGTAGGGGAACTTCTCCTGCTAGAATGGCT GGCAATGGCGGTGATGCTGCTCTTGCTTTGCTGCTGCTTGACAGATTG A | | | 145 | 46 |
| **E** | SARS-CoV-2 (NC_045512) | N gene | 486 | F3 | TCAAGGAACAACATTGCCAA | | 486 | 20 | 47 |
| | | | 771 | 83 | CTTCTTAGAAGCCTCAGCAG | | 771 | 20 | 48 |
| | | | | FIP(F1c+F2) | GCCAGCCATTCTAGCAGGAGGTAGTCGCANAGTTCAAGA | | | 40 | 49 |
| | | | | BIP(B1c+82) | AATGGCGGTGATGCTGCTTCTCAAGCTGGTTCAATCTG | | | 38 | 50 |
| | | | 604 | LoopF | TGCTGCCTGGAGTTGAATT | | 604 | 19 | 51 |
| | | | 656 | LoopB | TTGCTTTGCTGCTGCTTG | | 656 | 18 | 52 |
| | | | 566 | F2 | GTAGTCGCAACAGTTCAAGA | | 566 | 20 | 53 |
| | | | 636 | F1c | GCCAGCCATTCTAGCAGGAG | | 636 | 20 | 54 |
| | | | 694 | B2 | TCTCAAGCTGGTTCAATCTG | | 694 | 20 | 55 |
| | | | 637 | B1c | AATGGCGGTGATGCTGCT | | 637 | 18 | 56 |
| | | | | Product | GTAGTCGCAACAGTTCAAGAAATTCAACTCCAGGCAGCAGTAGGGGA ACTTCTCCTGCTAGAATGGCTGGCAATGGCGGTGATGCTGCTCTTGCT TTGCTGCTGCTTGACAGATTGAACCAGCTTGAGA | | | 129 | 57 |
| **F** | SARS-CoV-2 (NC_045512) | N gene | 538 | F3 | AGTCAAGCCTCTTCTCGT | | 538 | 18 | 58 |
| | | | 779 | 83 | TGCCGAGGCTTCTTAGAA | | 779 | 18 | 59 |
| | | | | FIP(F1c+F2) | AGCAGCATCACCGCCATTAATTCAACTCCAGGCAGC | | | 36 | 60 |
| | | | | BIP(B1c+82) | GCTTTGCTGCTGCTTGACAGTTGTTGGCCTTTACCAGAC | | | 39 | 61 |
| | | | 635 | LoopF | CCAGCCATTCTAGCAGGAG | | 635 | 19 | 62 |
| | | | 678 | LoopB | ATTGAACCAGCTTGAGAGCA | | 678 | 20 | 63 |
| | | | 586 | F2 | AATTCAACTCCAGGCAGC | | 586 | 18 | 64 |
| | | | 654 | F1c | AGCAGCATCACCGCCATT | | 654 | 18 | 65 |
| | | | 720 | B2 | TTGTTGGCCTTTACCAGAC | | 720 | 19 | 66 |
| | | | 658 | B1c | GCTTTGCTGCTGCTTGACAG | | 658 | 20 | 67 |
| | | | | Product | AATTCAACTCCAGGCAGCAGTAGGGGAACTTCTCCTGCTAGAATGGCT GGCAATGGCGGTGATGCTGCTCTTGCTTTGCTGCTGCTTGACAGATTG AACCAGCTTGAGAGCAAAATGTCTGGTAAAGGCCAACAA | | | 135 | 68 |
| **G** | SARS-CoV-2 (NC_045512) | N gene | 73 | F3 | GGCAGTAACCAGAATGGAG | | 73 | 19 | 69 |
| | | | 328 | B3 | AATACCATCTTGGACTGAGATC | | 328 | 22 | 70 |
| | | | | FIP(F1c+F2) | TTGGAACGCCTTGTCCTCGATACTGCGTCTTGGTTCAC | | | 38 | 71 |
| | | | | BIP(B1c+B2) | ACCAATAGCAGTCCAGATGACCCGTCACCACCACGAATTC | | | 40 | 72 |
| | | | 186 | LoopF | TTCCTTGCCATGTTGAGTGA | | 186 | 20 | 73 |
| | | | 249 | LoopB | AATTGGCTACTACCGAAGAGC | | 249 | 21 | 74 |
| | | | 143 | F2 | ATACTGCGTCTTGGTTCAC | | 143 | 19 | 75 |
| | | | 220 | F1c | TTGGAACGCCTTGTCCTCG | | 220 | 19 | 76 |
| | | | 295 | B2 | CGTCACCACCACGAATTC | | 295 | 18 | 77 |
| | | | 226 | B1c | ACCAATAGCAGTCCAGATGACC | | 226 | 22 | 78 |
| | | | | Product | ATACTGCGTCTTGGTTCACCGCTCTCACTCAACATGGCAAGGAAGACC TTAAATTCCCTCGAGGACAAGGCGTTCCAATTAACACCAATAGCAGTC CAGATGACCAAATTGGCTACTACCGAAGAGCTACCAGACGAATTCGTG GTGGTGACG | | | 153 | 79 |
| **H** | SARS-CoV-2 (NC_045512) | N gene | 208 | F3 | CAAGGCGTTCCAATTAACAC | | 208 | 20 | 80 |
| | | | 467 | B3 | GCAGCATTGITAGCAGGA | | 467 | 18 | 81 |
| | | | | FIP(F1c+F2) | ACCGTCACCACCACGAATTCCAATAGC AGTCCAGATGACC | | | 40 | 82 |
| | | | | BIP(B1c+B2) | CTAGGAACTGGGCCAGAAGCACCCATATGATGCCGTCT | | | 38 | 83 |
| | | | 269 | LoopF | GCTCTTCGGTAGTAGCCAATT | | 269 | 21 | 84 |
| | | | 359 | LoopB | GACTTCCCTATGGTGCTAACAA | | 359 | 22 | 85 |
| | | | 228 | F2 | CAATAGCAGTCCAGATGACC | | 228 | 20 | 86 |
| | | | 297 | F1c | ACCGTCACCACCACGAATTC | | 297 | 20 | 87 |
| | | | 398 | B2 | ACCCATATGATGCCGTCT | | 398 | 18 | 88 |
| | | | 337 | B1c | CTAGGAACTGGGCCAGAAGC | | 337 | 20 | 89 |
| | | | | Product | CAATAGCAGTCCAGATGACCAAATTGGCTACTACCGAAGAGCTACCAG ACGAATTCGTGGTGGTGACGGTAAAATGAAAGATCTCAGTCCAAGATG GTATTTCTACTACCTAGGAACTGGGCCAGAAGCTGGACTTCCCTATGG TGCTAACAAAGACGGCATCATATGGGT | | | 171 | 90 |
| **I** | SARS-CoV-2 (NC_045512) | N gene | 171 | F3 | TCAACATGGCAAGGAAGAC | | 171 | 19 | 91 |
| | | | 467 | B3 | GCAGCATTGITAGCAGGA | | 467 | 18 | 92 |
| | | | | FIP(F1c+F2) | ACCGTCACCACCACGAATTCTTAACACCAATAGCAGTCCAG | | | 41 | 93 |
| | | | | BIP(Blc+B2) | CTAGGAACTGGGCCAGAAGCACCCATATGATGCCGTCT | | | 38 | 94 |
| | | | 269 | LoopF | GCTCTTCGGTAGTAGCCAATT | | 269 | 21 | 95 |
| | | | 359 | LoopB | GACTTCCCTATGGTGCTAACAA | | 359 | 22 | 96 |
| | | | 221 | F2 | TTAACACCAATAGCAGTCCAG | | 221 | 21 | 97 |
| | | | 297 | F1c | ACCGTCACCACCACGAATTC | | 297 | 20 | 98 |
| | | | 398 | B2 | ACCCATATGATGCCGTCT | | 398 | 18 | 99 |
| | | | 337 | B1c | CTAGGAACTGGGCCAGAAGC | | 337 | 20 | 100 |
| | | | | Product | TTAACACCAATAGCAGTCCAGATGACCAAATTGGCTACTACCGAAGAG CTACCAGACGAATTCGTGGTGGTGACGGTAAAATGAAAGATCTCAGTC CAAGATGGTATTTCTACTACCTAGGAACTGGGCCAGAAGCTGGACTTC CCTATGGTGCTAACAAAGACGGCATCATATGGGT | | | 178 | 101 |
| **J** | SARS-CoV-2 (NC_045512) | N gene | 171 | F3 | TCAACATGGCAAGGAAGAC | | 171 | 19 | 102 |
| | | | 467 | B3 | GCAGCATTGTTAGCAGGA | | 467 | 18 | 103 |
| | | | | FIP(F1c+F2) | ACCGTCACCACCACGAATTCCCAATAGCAGTCCAGATGAC | | | 40 | 104 |
| | | | | BIP(B1c+82) | CTAGGAACTGGGCCAGAAGCACCCATATGATGCCGTCT | | | 38 | 105 |
| | | | 269 | LoopF | GCTCTTCGGTAGTAGCCAATT | | 269 | 21 | 106 |
| | | | 359 | LoopB | GACTTCCCTATGGTGCTAACAA | | 359 | 22 | 107 |
| | | | 227 | F2 | CCAATAGCAGTCCAGATGAC | | 227 | 20 | 108 |
| | | | 297 | F1c | ACCGTCACCACCACGAATTC | | 297 | 20 | 109 |
| | | | 398 | B2 | ACCCATATGATGCCGTCT | | 398 | 18 | 110 |
| | | | 337 | B1c | CTAGGAACTGGGCCAGAAGC | | 337 | 20 | 111 |
| | | | | Product | CCAATAGCAGTCCAGATGACCAAATTGGCTACTACCGAAGAGCTACCA GACGAATTCGTGGTGGTGACGGTAAAATGAAAGATCTCAGTCCAAGAT GGTATTTCTACTACCTAGGAACTGGGCCAGAAGCTGGACTTCCCTATG GTGCTAACAAAGACGGCATCATATGGGT | | | 172 | 112 |
| **K** | SARS-CoV-2 (NC_045512) | N gene | 208 | F3 | CAAGGCGTTCCAATTAACAC | | 208 | 20 | 113 |
| | | | 467 | B3 | GCAGCATTGTTAGCAGGA | | 467 | 18 | 114 |
| | | | | FIP(F1c+F2) | ACCGTCACCACCACGAATTCATAGCAGTCCAGATGACCA | | | 39 | 115 |
| | | | | BIP(B1c+82) | CTAGGAACTGGGCCAGAAGCACCCATATGATGCCGTCT | | | 38 | 116 |
| | | | 269 | LoopF | GCTCTTCGGTAGTAGCCAATT | | 269 | 21 | 117 |
| | | | 359 | LoopB | GACTTCCCTATGGTGCTAACAA | | 359 | 22 | 118 |
| | | | 230 | F2 | ATAGCAGTCCAGATGACCA | | 230 | 19 | 119 |
| | | | 297 | F1c | ACCGTCACCACCACGAATTC | | 297 | 20 | 120 |
| | | | 398 | B2 | ACCCATATGATGCCGTCT | | 398 | 18 | 121 |
| | | | 337 | B1c | CTAGGAACTGGGCCAGAAGC | | 337 | 20 | 122 |
| | | | | Product | ATAGCAGTCCAGATGACCAAATTGGCTACTACCGAAGAGCTACCAGAC GAATTCGTGGTGGTGACGGTAAAATGAAAGATCTCAGTCCAAGATGGT ATTTCTACTACCTAGGAACTGGGCCAGAAGCTGGACTTCCCTATGGTG CTAACAAAGACGGCATCATATGGGT | | | 169 | 123 |
| **L** | SARS-CoV-2 (NC_045512) | RdRp gene | 1 782 | F3 | TATGGTGGTTGGCACAAC | | 1 782 | 18 | 124 |
| | | | 2 057 | B3 | TGTGGCATCTCCTGATGA | | 2 057 | 18 | 125 |
| | | | | FIP(F1c+F2) | TGCGAGCAAGAACAAGTGTGGTGGGTTGGGATTATCCTAAATG | | | 43 | 126 |
| | | | | BIP(B1c+B2) | TETTGTAGLTTGTCACACCGTTCCACACATGACCATTTCACT | | | 42 | 127 |
| | | | 1 890 | LoopF | TAAGCATGTTAGGCATGGCT | | 1 890 | 20 | 128 |
| | | | 1 964 | LoopB | AGCTAATGAGTGTGCTCAAGT | | 1 964 | 21 | 129 |
| | | | 1 843 | F2 | TGGGTTGGGATTATCCTAAATG | | 1 843 | 22 | 130 |
| | | | 1 920 | F1c | TGCGAGCAAGAACAAGTGAGG | | 1 920 | 21 | 131 |
| | | | 2 008 | B2 | CCACACATGACCATTTCACT | | 2 008 | 20 | 132 |
| | | | 1 932 | B1c | TGTTGTAGCTTGTCACACCGTT | | 1 932 | 22 | 133 |
| | | | | Product | TGGGTTGGGATTATCCTAAATGTGATAGAGCCATGCCTAACATGCTTA GAATTATGGCCTCACTTGTTCTTGCTCGCAAACATACAACGTGTTGTAG CTTGTCACACCGTTTCTATAGATTAGCTAATGAGTGTGCTCAAGTATTG AGTGAAATGGTCATGTGTGG | | | 166 | 134 |
| **M** | SARS-CoV-2 (NC_045512) | RdRp gene | 1 782 | F3 | TATGGTGGTTGGCACAAC | | 1 782 | 18 | 135 |
| | | | 2 057 | B3 | TGTGGCATCTCCTGATGA | | 2 057 | 18 | 136 |
| | | | | FIP(F1c+F2) | TGCGAGCAAGAACAAGTGAGGGGGTTGGGATTATCCTAAATGT | | | 43 | 137 |
| | | | | BIP(B1c+B2) | TGTTGTAGCTTGTCACACCGTTCCACACATGACCATTTCACT | | | 42 | 138 |
| | | | 1 890 | LoopF | TAAGCATGTTAGGCATGGCT | | 1 890 | 20 | 139 |
| | | | 1 964 | LoopB | AGCTAATGAGTGTGCTCAAGT | | 1 964 | 21 | 140 |
| | | | 1 844 | F2 | GGGTTGGGATTATCCTAAATGT | | 1 844 | 22 | 141 |
| | | | 1 920 | F1c | TGCGAGCAAGAALAAGTGAGG | | 1 920 | 21 | 142 |
| | | | 2 008 | B2 | CCACACATGACCATITCACT | | 2 008 | 20 | 143 |
| | | | 1 932 | B1c | TGTTGTAGCTTGTCACACCGTT | | 1 932 | 22 | 144 |
| | | | | Product | GGGTTGGGATTATCCTAAATGTGATAGAGCCATGCCTAACATGCTTAG AATTATGGCCTCACTTGTTCTTGCTCGCAAACATACAACGTGTTGTAGC TTGTCACACCGTTTCTATAGATTAGCTAATGAGTGTGCTCAAGTATTGA GTGAAATGGTCATGTGTGG | | | 165 | 145 |
| **N** | SARS-CoV-2 (NC_045512) | RdRp gene | 1 899 | F3 | GCCTCACTTGTTCTTGCT | | 1 899 | 18 | 146 |
| | | | 2 161 | B3 | CGGACATACTTATCGGCAAT | | 2 161 | 20 | 147 |
| | | | | FIP(F1c+F2) | AACCGCCACACATGACCATTTTGTAGCTTGTCACCG | | | 38 | 148 |
| | | | | BIP(B1c+B2) | AGGTGGAACCTCATCAGGAGACCGTGACAGCTTGACAAA | | | 39 | 149 |
| | | | 1 993 | LoopF | TCACTCAATACTTGAGCACACT | | 1 993 | 22 | 150 |
| | | | 2 051 | LoopB | TGCCACAACTGCTTATGCTA | | 2 051 | 20 | 151 |
| | | | 1 934 | F2 | TTGTAGCTTGTCACACCG | | 1 934 | 18 | 152 |
| | | | 2 013 | F1c | AACCGCCACACATGACCATT | | 2 013 | 20 | 153 |
| | | | 2 103 | B2 | CCGTGACAGCTTGACAAA | | 2 103 | 18 | 154 |
| | | | 2 030 | B1c | AGGTGGAACCTCATCAGGAGA | | 2 030 | 21 | 155 |
| | | | | Product | TTGTAGCTTGTCACACCGTTTCTATAGATTAGCTAATGAGTGTGCTCAA GTATTGAGTGAAATGGTCATGTGTGGCGGTTCACTATATGTTAAACCA GGTGGAACCTCATCAGGAGATGCCACAACTGCTTATGCTAATAGTGTT TTTAACATTTGTCAAGCTGTCACGG | | | 170 | 156 |
| **O** | SARS-CoV-2 (NC_045512) | Rd Rp gene | 1 899 | F3 | GCCTCACTTGTTCTTGCT | | 1 899 | 18 | 157 |
| | | | 2 161 | B3 | CGGACATACTTATCGGCAAT | | 2 161 | 20 | 158 |
| | | | | FIP(Flc+F2) | CCGCCACACATGACCATTTCTGTTGTAGCTTGTCACACC | | | 39 | 159 |
| | | | | BIP(B1c+B2) | AGGTGGAACCTCATCAGGAGACCGTGACAGCTTGACAAA | | | 39 | 160 |
| | | | 1 991 | LoopF | ACTCAATACTTGAGCACACTCA | | 1 991 | 22 | 161 |
| | | | 2 051 | LoopB | TGCCACAACTGCTTATGCTA | | 2 051 | 20 | 162 |
| | | | 1 932 | F2 | TGTTGTAGCTTGTCACACC | | 1 932 | 19 | 163 |
| | | | 2 011 | F1c | CCGCCACACATGACCATTTC | | 2 011 | 20 | 164 |
| | | | 2 103 | 82 | CCGTGACAGCTTGACAAA | | 2 103 | 18 | 165 |
| | | | 2 030 | B1c | AGGTGGAACCTCATCAGGAGA | | 2 030 | 21 | 166 |
| | | | | Product | TGTTGTAGCTTGTCACACCGTTTCTATAGATTAGCTAATGAGTGTGCTC AAGTATTGAGTGAAATGGTCATGTGTGGCGGTTCACTATATGTTAAAC CAGGTGGAACCTCATCAGGAGATGCCACAACTGCTTATGCTAATAGTG TTTTTAACATTTGTCAAGCTGTCACGG | | | 172 | 167 |
| **P** | SARS-CoV-2 (NC_045512) | RdRp gene | 1 838 | F3 | CCTTATGGGTTGGGATTATCC | | 1 838 | 21 | 168 |
| | | | 2 114 | B3 | ATTAACATTGGCCGTGACA | | 2 114 | 19 | 169 |
| | | | | FIP(F1c+F2) | AACGGTGTGACAAGCTACAACAGCTTAGAATTATGGCCTCACT | | | 43 | 170 |
| | | | | BIP(B1c+B2) | GTCATGTGTGGCGGTTCACTAAAGCAGTTGTGGCATCTC | | | 39 | 171 |
| | | | 1 927 | LoopF | GTATGTTTGCGAGCAAGAACA | | 1 927 | 21 | 172 |
| | | | 2 028 | LoopB | CCAGGTGGAACCTCATCAG | | 2 028 | 19 | 173 |
| | | | 1 886 | F2 | GCTTAGAATTATGGLCTCACT | | 1 886 | 21 | 174 |
| | | | 1 953 | F1c | AACGGTGTGACAAGCTACAACA | | 1 953 | 22 | 175 |
| | | | 2 064 | B2 | AAGCAGTTGTGGCATCTC | | 2 064 | 18 | 176 |
| | | | 1 998 | B1c | GTCATGTGTGGCGGTTCACTA | | 1 998 | 21 | 177 |
| | | | | Product | GCTTAGAATTATGGCCTCACTTGTTCTTGCTCGCAAACATACAACGTGT TGTAGCTTGTCACACCGTTTCTATAGATTAGCTAATGAGTGTGCTCAAG TATTGAGTGAAATGGTCATGTGTGGCGGTTCACTATATGTTAAACCAG GTGGAACCTCATCAGGAGATGCCACAACTGCTT | | | 179 | 178 |
| **T** | SARS-CoV-2 (NC_045512) | RdRp gene | 1 838 | F3 | CCTTATGGGTTGGGATTATCC | | 1 838 | 21 | 179 |
| | | | 2 057 | B3 | TGTGGCATCTCCTGATGA | | 2 057 | 18 | 180 |
| | | | | FIP(F1c+F2) | CACGTTGTATGTTTGCGAGCAAGTGATAGAGCCATGCCTAAC | | | 42 | 181 |
| | | | | BIP(B1c+B2) | TGTAGCTIGTCACACCGTTTCTCCACACATGACCATITCACT | | | 42 | 182 |
| | | | 1 906 | LoopF | AGTGAGGCCATAATTCTAAGCA | | 1 906 | 22 | 183 |
| | | | 1 964 | LoopB | AGCTAATGAGTGTGCTCAAGT | | 1 964 | 21 | 184 |
| | | | 1 864 | F2 | GTGATAGAGCCATGCCTAAC | | 1 864 | 20 | 185 |
| | | | 1 933 | F1c | CACGTTGTATGTTTGCGAGCAA | | 1 933 | 22 | 186 |
| | | | 2 008 | B2 | CCACACATGACCATTTCACT | | 2 008 | 20 | 187 |
| | | | 1 935 | B1c | TGTAGCTTGTCACACCGTTTCT | | 1 935 | 22 | 188 |
| | | | | Product | GTGATAGAGCCATGCCTAACATGCTTAGAATTATGGCCTCACTTGTTCT TGCTCGCAAACATACAACGTGTTGTAGCTTGTCACACCGTTTCTATAGA TTAGCTAATGAGTGTGCTCAAGTATTGAGTGAAATGGTCATGTGTGG | | | 145 | 189 |
| **U** | SARS-CoV-2 (NC_045512) | RdRp gene | 1 838 | F3 | CCTTATGGGTTGGGATTATCC | | 1 838 | 21 | 190 |
| | | | 2 057 | B3 | TGTGGCATCTCCTGATGA | | 2 057 | 18 | 191 |
| | | | | FIP(F1c+F2) | CACGTTGTATGTTTGCGAGCAAAATGTGATAGAGCCATGCC | | | 41 | 192 |
| | | | | BIP(B1c+B2) | TGTAGCTTGTCACACCGTTTCTCCACACATGACCATTTCACT | | | 42 | 193 |
| | | | 1 906 | LoopF | AGTGAGGCCATAATTCTAAGCA | | 1 906 | 22 | 194 |
| | | | 1 964 | LoopB | AGCTAATGAGTGTGCTCAAGT | | 1 964 | 21 | 195 |
| | | | 1 861 | F2 | AATGTGATAGAGCCATGCC | | 1 861 | 19 | 196 |
| | | | 1 933 | F1c | CACGTTGTATGTTTGCGAGCAA | | 1 933 | 22 | 197 |
| | | | 2 008 | B2 | CCACACATGACCATTTCACT | | 2 008 | 20 | 198 |
| | | | 1 935 | B1c | TGTAGCTTGTCACACCGTTTCT | | 1 935 | 22 | 199 |
| | | | | Product | AATGTGATAGAGCCATGCCTAACATGCTTAGAATTATGGCCTCACTTG TTCTTGCTCGCAAACATACAACGTGTTGTAGCTTGTCACACCGTTTCTA TAGATTAGCTAATGAGTGTGCTCAAGTATTGAGTGAAATGGTCATGTG TGG | | | 148 | 200 |
| **V** | SARS-CoV-2 (NC_045512) | RdRp gene | 1 838 | F3 | CCTTATGGGTTGGGATTATCC | | 1 838 | 21 | 201 |
| | | | 2 057 | 83 | TGTGGCATCTCCTGATGA | | 2 057 | 18 | 202 |
| | | | | FIP(F1c+F2) | CACGTTGTATGTTTGCGAGCAAATGTGATAGAGCCATGCCTA | | | 42 | 203 |
| | | | | BIP(B1c+B2) | TGTAGCTTGICACACCGTTTCTCCACACATGACCATITCACT | | | 42 | 204 |
| | | | 1 906 | LoopF | AGTGAGGCCATAATTCTAAGCA | | 1 906 | 22 | 205 |
| | | | 1 964 | LoopB | AGCTAATGAGTGTGCTCAAGT | | 1 964 | 21 | 206 |
| | | | 1 862 | F2 | ATGTGATAGAGCCATGCCTA | | 1 862 | 20 | 207 |
| | | | 1 933 | F1c | CACGTTGTATGTTTGCGAGCAA | | 1 933 | 22 | 208 |
| | | | 2 008 | B2 | CCACACATGACCATTTCACT | | 2 008 | 20 | 209 |
| | | | 1 935 | B1c | TGTAGCTTGTCACACCGTTTCT | | 1 935 | 22 | 210 |
| | | | | Product | ATGTGATAGAGCCATGCCTAACATGCTTAGAATTATGGCCTCACTTGTT CTTGCTCGCAAACATACAACGTGTTGTAGCTTGTCACACCGTTTCTATA GATTAGCTAATGAGTGTGCTCAAGTATTGAGTGAAATGGTCATGTGTG G | | | 147 | 211 |
| **AC** | SARS-CoV-2 (NC_045512) | ORF3a + E | 610 | F3 | CACAGTTACTTCACTTCAGACT | | 610 | 22 | 212 |
| | | | 960 | B3 | CGCAGTAAGGA1GGCTAG | | 960 | 18 | 213 |
| | | | | FIP(F1c+F2) | GCTAGTAGTCGTCGTCGGTTCTGATGAGCCTGAAGAACATG | | | 41 | 214 |
| | | | | BIP(B1c+B2) | AAGCACAAGCTGATGAGTACGAGTAACTAGCAAGAATACCACGA | | | 44 | 215 |
| | | | 759 | LoopF | GGATGAACCGTCGATTGTGT | | 759 | 20 | 216 |
| | | | 867 | LoopB | TTCGGAAGAGACAGGTACG | | 867 | 19 | 217 |
| | | | 711 | F2 | TGATGAGCCTGAAGAACATG | | 711 | 20 | 218 |
| | | | 816 | F1c | GCTAGTAGTCGTCGTCGGTTC | | 816 | 21 | 219 |
| | | | 941 | 82 | GTAACTAGCAAGAATACCACGA | | 941 | 22 | 220 |
| | | | 827 | B1c | AAGCACAAGCTGATGAGTACGA | | 827 | 22 | 221 |
| | | | | Product | TGATGAGCCTGAAGAACATGTCCAAATTCACACAATCGACGGTTCATC CGGAGTTGTTAATCCAGTAATGGAACCAATTTATGATGAACCGACGAC GACTACTAGCGTGCCTTTGTAAGCACAAGCTGATGAGTACGAACTTAT GTACTCATTCGTTTCGGAAGAGACAGGTACGTTAATAGTTAATAGCGT ACTTCTTTTTCTTGCTTTCGTGGTATTCTTGCTAGTTAC | | | 231 | 222 |

### References

1. Rapid Detection of Novel Coronavirus (COVID-19) by Reverse Transcription-Loop-Mediated Isothermal Amplification. Laura E Lamb, Sarah N Bartolone, Elijah Ward, Michael B Chancellor. medRxiv 2020.02.19.20025155; doi: https://doi.org/10.1101/2020.02.19.20025155
2. Rapid colorimetric detection of COVID-19 coronavirus using a reverse tran-scriptional loop-mediated isothermal amplification (RT-LAMP) diagnostic plat-form: iLACO. Lin Yu, Shanshan Wu, Xiaowen Hao, Xuelong Li, Xiyang Liu, Shenglong Ye, Heng Han, Xue Dong, Xin Li, Jiyao Li, Jianmin Liu, Na Liu, Wanzhong Zhang, Vicent Pelechano, Wei-Hua Chen, Xiushan Yin. medRxiv 2020.02.20.20025874; doi: https://doi.org/10.1101/2020.02.20.20025874.
3. SalivaDirect: A simplified and flexible platform to enhance SARS-CoV-2 testing capacity. Chantal B.F. Vogels, Anne E. Watkins, Christina A. Harden, Doug E. Brackney, Jared Shafer, Jianhui Wang, César Caraballo, Chaney C. Kalinich, Isabel M. Ott, Joseph R. Fauver, Eriko Kudo, Peiwen Lu, Arvind Venkataraman, Maria Tokuyama, Adam J. Moore, M. Catherine Muenker, Arnau Casanovas-Massana, John Fournier, Santos Bermejo, Melissa Campbell, Rupak Datta, Allison Nelson, Kelly Anastasio, Michael H. Askenase, Maria Batsu, Sean Bickerton, Kristina Brower, Molly L. Bucklin, Staci Cahill, Yiyun Cao, Edward Courchaine, Giuseppe DeIuliis, Rebecca Earnest, Bertie Geng, Benjamin Goldman-Israelow, Ryan Handoko, William Khoury-Hanold, Daniel Kim, Lynda Knaggs, Maxine Kuang, Sarah Lapidus, Joseph Lim, Melissa Linehan, Alice Lu-Culligan, Anjelica Martin, Irene Matos, David McDonald, Maksym Minasyan, Maura Nakahata, Nida Naushad, Jessica Nouws, Abeer Obaid, Camila Odio, Ji Eun Oh, Saad Omer, Annsea Park, Hong-Jai Park, Xiaohua Peng, Mary Petrone, Sarah Prophet, Tyler Rice, Kadi-Ann Rose, Lorenzo Sewanan, Lokesh Sharma, Albert C. Shaw, Denise Shepard, Mikhail Smolgovsky, Nicole Sonnert, Yvette Strong, Codruta Todeasa, Jordan Valdez, Sofia Velazquez, Pavithra Vijayakumar, Elizabeth B. White, Yexin Yang, Charles S. Dela Cruz, Albert I. Ko, Akiko Iwasaki, Harlan M. Krumholz, J.D. Matheus, Pei Hui, Chen Liu, Shelli F. Farhadian, Robby Sikka, Anne L. Wyllie, Nathan D. Grubaugh, Med 2021, 2(3):263-280. https://doi.org/10.1016/j.medj.2020.12.010.

## Claims

1. A first set of oligonucleotide primers for isothermal nucleic acid amplification of Severe acute respiratory syndrome coronavirus 2 (SARS-CoV-2) RNA, which targets SARS-CoV-2 Nucleocapsid (N) gene of SEQ ID NO: 1 and is a set E comprising primers consisting of sequences SEQ ID NO: 47 to 52.

2. The first set of primers according to claim 1, which is specific for SARS-CoV-2.

3. The first set of oligonucleotide primers according to claim 1 or claim 2, which comprise at least one labelled oligonucleotide primer.

4. The first set of oligonucleotide primers according to claim 3, wherein the oligonucleotide primer is labelled at the 5' end.

5. The first set of oligonucleotide primers according to claim 3 or claim 4, which comprises a fluorescent oligonucleotide primer.

6. The first set of oligonucleotide primers according to claim 5, wherein the fluorescent oligonucleotide primer is chosen from SEQ ID NO: 49 and SEQ ID NO: 50; preferably SEQ ID NO: 50.

7. A combination of oligonucleotide primers comprising at least a first set of primers specific for SARS-CoV-2 according to any one of claims 1 to 6 and a second set of primers specific for Betacoronavirus group B/C, wherein the first set of primers comprises the sequences SEQ ID NO: 47 to 52 which hybridize to said N gene sequence or complement thereof, and the second set of primers comprises the sequences SEQ ID NO: 179 to 184 which hybridize to the RNA-dependent RNA polymerase (RdRp) gene sequence of SEQ ID NO: 2 or complement thereof.

8. The combination of claim 7, which provides a differential diagnosis between infection and disease caused by SARS-CoV-2 and other Betacoronavirus group B/C such as SARS-CoV and MERS-CoV.

9. The combination of claim 7, wherein the first set of primers or the second set of primers comprise(s) at least one labelled oligonucleotide primer.

10. A kit for the detection of SARS-CoV-2 and/or Betacoronavirus group B/C RNA, comprising at least one set of oligonucleotide primers according to any one of claims 1 to 6 or a combination thereof according to any one of claims 7 to 9.

11. The kit according to claim 10, which further comprises at least one short quenching probe complementary to a Forward or Backward inner primer for isothermal nucleic acid amplification of SARS-CoV-2 RNA.

12. The kit according to claim 11, wherein the short complementary quenching probe comprises a sequence selected from the group consisting of: 5'-CCGCCATT-3', 5'-TGGCTGGC-3', 5'- ACAAGCTACA -3' (SEQ ID NO: 225) and 5'- ATACAACGTG - 3'(SEQ ID NO: 226); and/or wherein the short complementary quenching probe is labelled at the 3' end with a black hole quencher.

13. Use of the kit according to any one of claims 10 to 12, for the *in vitro* diagnosis of SARS-CoV-2 and/or Betacoronavirus group B/C infection and associated disease in a biological sample from a subject.

14. Use of the kit according to any one of claims 10 to 12, for the *in vitro* detection of a contamination with SARS-CoV-2 and/or Betacoronavirus group B/C in an environmental sample.

15. A method of detection of SARS-CoV-2 and/or Betacoronavirus group B/C RNA in a sample, comprising:
- subjecting said sample to an isothermal nucleic acid amplification reaction using a set of primers for amplifying a target sequence of said viral RNA according to claim 1 to 6, or a combination thereof according to claim 7 or claim 8, and
- detecting the presence of an amplification product for said target sequence.

16. The set of oligonucleotide primers, combination, kit, method or use according to any one of claims 1 to 15, wherein said SARS-CoV-2 is selected from the group consisting of: isolate Wuhan-Hu-1 and variants thereof such as alpha, beta, gamma, delta variants and other variants.

## Patentansprüche

1. Ein erster Satz von Oligonukleotidprimern für die isotherme Nukleinsäureamplifikation von RNA des schweren akuten respiratorischen Syndroms Coronavirus 2 (SARS-CoV-2), der auf das SARS-CoV-2-Nukleokapsid (N)-Gen von SEQ ID NO: 1 abzielt und ein Satz E ist, der Primer umfasst, die aus den Sequenzen SEQ- -ID NO: 47 bis 52 bestehen.

2. Der erste Satz von Primern gemäß Anspruch 1, der spezifisch für SARS-CoV-2 ist.

3. Der erste Satz von Oligonukleotidprimern gemäß Anspruch 1 oder Anspruch 2, der mindestens einen markierten Oligonukleotidprimer umfasst.

4. Der erste Satz von Oligonukleotidprimern gemäß Anspruch 3, wobei der Oligonukleotidprimer am 5'-Ende markiert ist.

5. Der erste Satz von Oligonukleotidprimern gemäß Anspruch 3 oder Anspruch 4, der einen fluoreszierenden Oligonukleotidprimer umfasst.

6. Der erste Satz von Oligonukleotidprimern gemäß Anspruch 5, wobei der fluoreszierende Oligonukleotidprimer aus SEQ ID NO: 49 und SEQ ID NO: 50 ausgewählt ist, vorzugsweise SEQ ID NO: 50.

7. Eine Kombination von Oligonukleotidprimern, die mindestens einen ersten Satz von Primern, die gemäß einem der Ansprüche 1 bis 6 für SARS-CoV-2 spezifisch sind, und einen zweiten Satz von Primern, die für Betacoronavirus-Gruppe B/C spezifisch sind, umfasst, wobei der erste Satz von Primern die Sequenzen SEQ ID NO: 47 bis 52 umfasst, die an die N-Gensequenz oder deren Komplement hybridisieren, und der zweite Satz von Primern die Sequenzen SEQ ID NO: 179 bis 184 umfasst, die an die RNA-abhängige RNA-Polymerase (RdRp)-Gensequenz von SEQ ID NO: 2 oder deren Komplement hybridisieren.

8. Die Kombination aus Anspruch 7, die eine Differentialdiagnose zwischen einer Infektion und einer Erkrankung durch SARS-CoV-2 und andere Betacoronavirus-Gruppe B/C wie SARS-CoV und MERS-CoV ermöglicht.

9. Die Kombination aus Anspruch 7, wobei der erste Satz von Primern oder der zweite Satz von Primern mindestens einen markierten Oligonukleotidprimer umfasst.

10. Kit zum Nachweis von SARS-CoV-2 und/oder Betacoronavirus der Gruppe B/C-RNA, umfassend mindestens einen Satz von Oligonukleotidprimern gemäß einem der Ansprüche 1 bis 6 oder eine Kombination davon gemäß einem der Ansprüche 7 bis 9.

11. Der Kit gemäß Anspruch 10, der ferner mindestens eine kurze Quenching-Sonde umfasst, die zu einem Vorwärts- oder Rückwärts-Innenprimer für die isotherme Nukleinsäureamplifikation von SARS-CoV-2-RNA komplementär ist.

12. Das Kit gemäß Anspruch 11, wobei die kurze komplementäre Quenching-Sonde eine Sequenz umfasst, die aus der Gruppe ausgewählt ist, bestehend aus: 5'-CCGCCATT-3', 5'-TGGCTGGC-3', 5'- ACAAGCTACA -3' (SEQ ID NO: 225) und 5'-ATACAACGTG-3' (SEQ ID NO: 226); und/oder wobei die kurze komplementäre Quenching-Sonde am 3'-Ende mit einem Black-Hole-Quencher markiert ist.

13. Verwendung des Kits gemäß einem der Ansprüche 10 bis 12 zur In-vitro-Diagnose einer SARS-CoV-2- und/oder Betacoronavirus-Gruppe-B/C-Infektion und einer damit verbundenen Erkrankung in einer biologischen Probe von einem Probanden.

14. Verwendung des Kits gemäß einem der Ansprüche 10 bis 12 zum In-vitro-Nachweis einer Kontamination mit SARS-CoV-2 und/oder Betacoronavirus der Gruppe B/C in einer Umweltprobe.

15. Verfahren zum Nachweis von SARS-CoV-2 und/oder Betacoronavirus der Gruppe B/C RNA in einer Probe, umfassend:
- Unterziehen der Probe einer isothermen Nukleinsäureamplifikationsreaktion unter Verwendung eines Satzes von Primern zum Amplifizieren einer Zielsequenz der viralen RNA gemäß Anspruch 1 bis 6 oder einer Kombination davon gemäß Anspruch 7 oder Anspruch 8, und
- Nachweisen des Vorhandenseins eines Amplifikationsprodukts für die Zielsequenz.

16. Der Satz von Oligonukleotidprimern, die Kombination, das Kit, das Verfahren oder die Verwendung gemäß einem der Ansprüche 1 bis 15, wobei das SARS-CoV-2 aus der Gruppe ausgewählt ist, die aus dem Isolat Wuhan-Hu-1 und Varianten davon, wie Alpha-, Beta-, Gamma-, Delta-Varianten und anderen Varianten, besteht.

## Revendications

1. Un premier ensemble d'amorces oligonucléotidiques pour l'amplification isotherme de l'acide nucléique de l'ARN du coronavirus 2 responsable du syndrome respiratoire aigu sévère (SARS-CoV-2), qui cible le gène de la nucléocapside (N) du SARS-CoV-2 de SEQ ID NO : 1 et est un ensemble E comprenant des amorces constituées des séquences SEQ ID NO : 47 à 52.

2. Le premier ensemble d'amorces selon la revendication 1, qui est spécifique du SARS-CoV-2.

3. Le premier ensemble d'amorces oligonucléotidiques selon la revendication 1 ou la revendication 2, comprenant au moins une amorce oligonucléotidique marquée.

4. Le premier ensemble d'amorces oligonucléotidiques selon la revendication 3, dans lequel l'amorce oligonucléotidique est marquée à l'extrémité 5'.

5. Le premier ensemble d'amorces oligonucléotidiques selon la revendication 3 ou la revendication 4, comprenant une amorce oligonucléotidique fluorescente.

6. Le premier ensemble d'amorces oligonucléotidiques selon la revendication 5, dans lequel l'amorce oligonucléotidique fluorescente est choisie parmi SEQ ID NO : 49 et SEQ ID NO : 50 ; de préférence SEQ ID NO : 50.

7. Combinaison d'amorces oligonucléotidiques comprenant au moins un premier ensemble d'amorces spécifiques du SARS-CoV-2 selon l'une des revendications 1 à 6 et un deuxième ensemble d'amorces spécifiques du groupe B/C des Betacoronavirus, dans laquelle le premier ensemble d'amorces comprend les séquences SEQ ID NO : 47 à 52 qui s'hybrident à ladite séquence du gène N ou à son complément, et le second ensemble d'amorces comprend les séquences SEQ ID NO : 179 à 184 qui s'hybrident à la séquence du gène SEQ ID NO : 2 de l'ARN polymérase ARN-dépendante (RdRp) ou à son complément.

8. La combinaison de la revendication 7, qui permet un diagnostic différentiel entre l'infection et la maladie causées par le SARS-CoV-2 et d'autres Betacoronavirus du groupe B/C tels que le SARS-CoV et le MERS-CoV.

9. La combinaison de la revendication 7, dans laquelle le premier ensemble d'amorces ou le deuxième ensemble d'amorces comprend au moins une amorce oligonucléotidique marquée.

10. Un kit pour la détection de l'ARN du SARS-CoV-2 et/ou des Betacoronavirus du groupe B/C, comprenant au moins un ensemble d'amorces oligonucléotidiques selon l'une des revendications 1 à 6 ou une combinaison de celles-ci selon l'une des revendications 7 à 9.

11. Le kit selon la revendication 10, qui comprend en outre au moins une courte sonde d'extinction complémentaire d'une amorce interne en avant ou en arrière pour l'amplification isotherme de l'acide nucléique de l'ARN du SARS-CoV-2.

12. Le kit selon la revendication 11, dans lequel la courte sonde d'extinction complémentaire comprend une séquence choisie dans le groupe constitué de : 5'-CCGCCATT-3', 5'-TGGCTGGC-3', 5'- ACAAGCTACA -3' (SEQ ID NO : 225) et 5'-ATACAACGTG -3' (SEQ ID NO : 226) ; et/ou dans lequel la courte sonde d'extinction complémentaire est marquée à l'extrémité 3' avec un suppresseur de trou noir.

13. Utilisation du kit selon l'une des revendications 10 à 12 pour le diagnostic *in vitro* de l'infection par le SARS-CoV-2 et/ou le Betacoronavirus du groupe B/C et de la maladie associée dans un échantillon biologique prélevé sur un sujet.

14. Utilisation du kit selon l'une des revendications 10 à 12, pour la détection *in vitro* d'une contamination par le SARS-CoV-2 et/ou le Betacoronavirus du groupe B/C dans un échantillon environnemental.

15. Méthode de détection de l'ARN du SARS-CoV-2 et/ou du Betacoronavirus du groupe B/C dans un échantillon, comprenant :
- une soumission dudit échantillon à une réaction isotherme d'amplification de l'acide nucléique utilisant un ensemble d'amorces pour amplifier une séquence cible dudit ARN viral selon les revendications 1 à 6, ou une combinaison de celles-ci selon la revendication 7 ou la revendication 8, et
- une détection de la présence d'un produit d'amplification pour ladite séquence cible.

16. Ensemble d'amorces oligonucléotidiques, combinaison, kit, méthode ou utilisation selon l'une des revendications 1 à 15, dans lequel ledit SARS-CoV-2 est choisi dans le groupe comprenant : l'isolat Wuhan-Hu-1 et ses variants tels que les variants alpha, bêta, gamma, delta et autres variants.
